(19) Europäisches Patentamt
European Patent Office
Office européen des brevets

(11) Veröffentlichungsnummer: **0 165 422**
**B1**

# (12) EUROPÄISCHE PATENTSCHRIFT

(45) Veröffentlichungstag der Patentschrift: **09.03.88**

(51) Int. Cl.⁴: **C 07 C 147/10**, A 61 K 31/00

(21) Anmeldenummer: **85105478.3**

(22) Anmeldetag: **06.05.85**

(54) **Neue substituierte Bis(4-aminophenyl)sulfone, ihre Herstellung und ihre Verwendung als Arzneimittel.**

(30) Priorität: **22.05.84 DE 3419009**

(43) Veröffentlichungstag der Anmeldung: **27.12.85 Patentblatt 85/52**

(45) Bekanntmachung des Hinweises auf die Patenterteilung: **09.03.88 Patentblatt 88/10**

(84) Benannte Vertragsstaaten: **AT BE CH DE FR GB IT LI LU NL SE**

(56) Entgegenhaltungen:
**GB - A - 1 330 609**
**US - A - 2 589 211**
**US - A - 2 623 063**
**US - A - 4 061 791**

(73) Patentinhaber: **Dr. Karl Thomae GmbH, Postfach 1755, D-7950 Biberach (Riss) (DE)**

(72) Erfinder: **Seydel, Joachim, Prof. Dr. Dipl.-Chem., Mühloh 2, D-2061 Borstel (DE)**
Erfinder: **Pieper, Helmut, Dr. Dipl.-Chem., Kapellenweg 5, D-7950 Biberach 1 (DE)**
Erfinder: **Krüger, Gerd, Dr. Dipl.-Chem., Ginsterhalde 30, D-7950 Biberach 1 (DE)**
Erfinder: **Noll, Klaus, Dr. Dipl.-Chem., Im Schönblick 3, D-7951 Warthausen (DE)**
Erfinder: **Keck, Johannes, Dr. Dipl.-Chem., Talfeldstrasse 27, D-7950 Biberach 1 (DE)**
Erfinder: **Lechner, Uwe, Dr., Panoramastrasse 12, D-7951 Ummendorf (DE)**

**EP 0 165 422 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

In der US-A-2 385 899 wird die Verbindung Bis-(4-aminophenyl)-sulfon beschrieben, welche eine Hemmwirkung auf das Wachstum von Bakterien, Mykobakterien und Plasmodien aufweist.

Es wurde nun gefunden, dass die neuen substituierten Bis-(4-aminophenyl)-sulfone der allgemeinen Formel

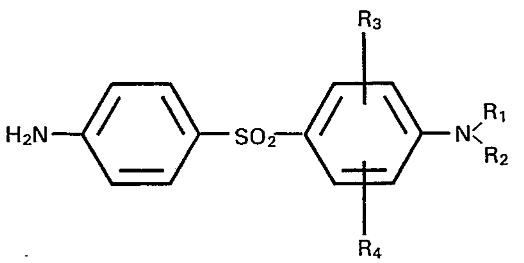

(I)

und deren Säureadditionssalze, insbesondere deren physiologisch verträgliche Säureadditionssalze, überlegene biologische Eigenschaften aufweisen, insbesondere hinsichtlich ihrer Verträglichkeit und ihrer Hemmwirkung gegenüber Bakterien, Mykobakterien und Plasmodien.

In der obigen allgemeinen Formel I bedeutet

$R_1$ ein Wasserstoffatom, eine Alkylgruppe mit 1 bis 7 Kohlenstoffatomen oder eine Cycloalkylgruppe mit 3 bis 7 Kohlenstoffatomen,

$R_2$ ein Wasserstoffatom oder eine Alkylgruppe mit 1 bis 3 Kohlenstoffatomen,

$R_3$ eine Nitril-, Alkylaminocarbonyl-, Dialkylaminocarbonyl-, N-Cycloalkyl-alkylaminocarbonyl-, Alkylamino-, Dialkylamino-, Dialkylaminocarbonylalkoxy-, Alkylaminosulfonyl-, Dialkylaminosulfonyl-, Hydroxyalkyl-, Alkylcarbonyl-, Aminoalkyl- oder Alkoxyalkylgruppe

oder, wenn $R_1$ und $R_2$ jeweils ein Wasserstoffatom darstellen, auch eine Hydroxy- oder Hydroxycarbonyl-alkoxygruppe

oder, wenn $R_1$ eine Alkyl- oder Cycloalkylgruppe und $R_2$ ein Wasserstoffatom oder eine Alkylgruppe darstellen, auch ein Halogenatom, eine Trifluormethyl-, Nitro-, Amino-, Aminosulfonyl-, Aminocarbonyl-, Alkyl-, Carboxy- oder Alkoxycarbonylgruppe und

$R_4$ ein Wasserstoffatom oder, wenn $R_1$ und $R_2$ jeweils ein Wasserstoffatom und $R_3$ in 2-Stellung ein Halogenatom oder eine Hydroxygruppe darstellen, auch ein Halogenatom, eine Hydroxy- oder Alkoxygruppe,

wobei die bei der Definition der Reste $R_3$ und $R_4$ vorstehend erwähnten Alkylteile jeweils 1 bis 3 Kohlenstoffatome und der Cycloalkylteil 3 bis 7 Kohlenstoffatome enthalten können.

Gegenstand der vorliegenden Erfindung sind somit die neuen Verbindungen der obigen allgemeinen Formel I, deren Säureadditionssalze, insbesondere deren Säureadditionssalze mit physiologisch verträglichen anorganischen oder organischen Säuren, diese Verbindungen bzw. ihre physiologisch verträgliche Säureadditionssalze enthaltende Arzneimittel und ihre Herstellung.

Ein weiterer Gegenstand der vorliegenden Erfindung sind die neuen Kombinationen der neuen substituierten Bis-(4-aminophenyl)-sulfone der obigen allgemeinen Formel I sowie deren physiologisch verträglichen Säureadditionssalze mit einem Dihydrofolsäure-Reduktase-Hemmer wie Pyrimethamin, Trimethoprim oder Trimethoprim-Derivaten.

Beispielsweise kommt für die bei der Definition der Reste $R_1$ bis $R_4$ eingangs erwähnten Bedeutungen

für $R_1$ die des Wasserstoffatoms, der Methyl-, Äthyl-, n-Propyl-, Isopropyl-, n-Butyl-, Isobutyl-, n-Pentyl-, Isoamyl-, n-Hexyl-, N-Heptyl-, Cyclobutyl-, Cyclopentyl-, Cyclohehyl- oder Cycloheptylgruppe,

für $R_2$ die des Wasserstoffatoms, der Methyl-, Äthyl-, n-Propyl- oder Isopropylgruppe,

für $R_3$ die der Cyano-, Methylaminocarbonyl-Äthylaminocarbonyl-, Isopropylaminocarbonyl-, Dimethylaminocarbonyl-, Diäthylaminocarbonyl-, D-n-propylaminocarbonyl-, N-Methyl-äthylaminocarbonyl-, N-Methyl-cyclopentylaminocarbonyl-, N-Methyl-cyclohexylaminocarbonyl-, N-Methyl-cyclopentyl-aminocarbonyl-, N-Äthyl-cyclohexylaminocarbonyl-, Methylamino-, Äthylamino-, n-Propylamino-, Isopropylamino-, Dimethylamino-, Diäthylamino-, Diisopropylamino-, N-Methyläthylamino-, N-Äthyl-n-propylamino-, Dimethylaminocarbonylmethoxy-, Diäthylaminocarbonylmethoxy, 2-Dimethylaminocarbonyläthoxy, 2-Diäthylaminocarbonyläthoxy-, Methylaminosulfonyl-, Äthylaminosulfonyl-, Isopropylaminosulfonyl-, Dimethylaminosulfonyl-, Diäthylaminosulfonyl-, Hydroxymethyl-, 1-Hydroxyäthyl-, 2-Hydroxyäthyl-, 1-Hydroxypropyl-, 3-Hydroxypropyl-, Methylcarbonyl-, Äthylcarbonyl-, n-Propylcarbonyl-, Aminomethyl-, 2-Aminoäthyl-, 3-Aminopropyl-, Methoxymethyl-, 2-Methoxyäthyl- Äthoxymethyl-, 2-Äthoxyäthyl-, n-Propoxymethyl-, 2-n-Propoxyäthyl-, Hydroxy-, Hydroxycarbonylmethoxy-, 2-Hydroxycarbonyläthoxy-, 3-Hydroxycarbonylpropoxy-, 2-Dimethylamino-äthoxy-, 2-Diäthylaminoäthoxy-, Trifluormethyl-, Nitro-, Amino-, Aminosulfonyl-, Aminocarbonyl-, Methyl-, Äthyl-, n-Propyl-, Isopropyl-, Carboxy-, Methoxycarbonyl-, Äthoxycarbonyl-, n-Prooxycarbonyl- oder Isopropoxycarbonylgruppe oder die des Fluor-, Chlor-, Brom- oder Jodatoms und

für $R_4$ die des Wasserstoff-, Fluor-, Chlor-, Brom- oder Jodatoms, der Hydroxy-, Methoxy-, Äthoxy- oder n-Propoxygruppe in Betracht.

Bevorzugte Verbindungen der obigen allgemeinen Formel I sind diejenigen, in denen

$R_1$ ein Wasserstoffatom, eine Alkylgruppe mit 1 bis 7 Kohlenstoffatomen oder eine Cycloalkylgruppe mit 4 bis 7 Kohlenstoffatomen,

$R_2$ ein Wasserstoffatom oder eine Methylgruppe,

$R_3$ eine Nitril-, Methylaminocarbonyl-, N-Cyclohexyl-methyl-aminocarbonyl-, Methylamino-, Dimethylamino-, Dimethylaminocarbonylmethoxy-, Hydroxymethyl-, Hydroxyäthyl-, Methylcarbonyl-, Aminocarbonyl- oder Methoxymethylgruppe

oder, wenn $R_1$ und $R_2$ jeweils ein Wasserstoffatom darstellen, auch eine Hydroxy oder Hydroxycarbonylmethoxygruppe

oder, wenn $R_1$ eine Alkyl- oder Cycloalkylgruppe

und $R_2$ ein Wasserstoffatom oder eine Methylgruppe darstellen, auch ein Chlor- oder Bromatom, eine Methyl- Trifluormethyl-, Nitro-, Amino- oder Aminocarbonylgruppe und

$R_4$ ein Wasserstoffatom oder, wenn $R_1$ und $R_2$ jeweils ein Wasserstoffatom und $R_3$ in 2-Stellung eine Hydroxygruppe, ein Chlor- oder Bromatom darstellen, auch ein Chlor- oder Bromatom, eine Hydroxy- oder Methoxygruppe bedeuten.

Besonders bevorzugte Verbindungen der obigen allgemeinen Formel I sind jedoch diejenigen, in denen

$R_1$ ein Wasserstoffatom, eine Alkylgruppe mit 1 bis 3 Kohlenstoffatomen oder eine Cycloalkylgruppe mit 4 bis 7 Kohlenstoffatomen,

$R_2$ ein Wasserstoffatom oder, wenn $R_1$ eine Methylgruppe darstellt, auch eine Methylgruppe,

$R_3$ in 2-Stellung ein Chlor- oder Bromatom, eine Methyl-, Trifluormethyl- Hydroxymethyl-, Hydroxyäthyl-, Methylamino-, Dimethylamino-, Cyano- oder Methylcarbonylgruppe und

$R_4$ ein Wasserstoffatom bedeuten.

Erfindungsgemäss erhält man die neuen Verbindungen nach folgenden Verfahren:

a.) Reduktion einer Verbindung der allgemeinen Formel

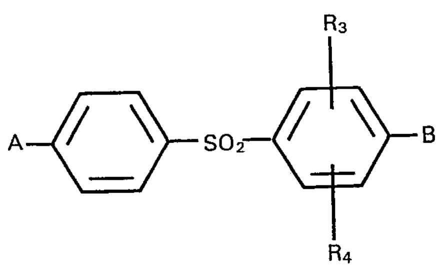

(II)

in der

$R_3$ und $R_4$ wie eingangs definiert sind,

einer der Reste A oder B eine Nitrogruppe und der andere der Reste A oder B eine $\genfrac{}{}{0pt}{}{R_1}{R_2}\!>$N-Gruppe, wobei $R_1$ und $R_2$ wie eingangs definiert sind, oder ebenfalls eine Nitrogruppe darstellt.

Die Reduktion wird zweckmässigerweise in einem Lösungsmittel oder Lösungsmittelgemisch wie Wasser, Methanol, Äthanol, Eisessig, Essigsäureäthylester, Dimethylformamid, Wasser/Äthanol oder Wasser/Tetrahydrofuran in Gegenwart eines Reduktionsmittels, z.B. mit Wasserstoff in Gegenwart eines Hydrierungskatalysators wie Raney-Nickel, Platin oder Palladium/Kohle, mit Metallen wie Eisen, Zinn oder Zink in Gegenwart einer Säure wie Salzsäure oder Essigssäure, mit Salzen wie Eisen(II)sulfat, Zinn(II)chlorid/Salzsäure oder Natriumdithionit in Gegenwart einer Base wie Natronlauge oder Pyridin oder mit Hydrazin in Gegenwart von Raney-Nickel, bei Temperaturen zwischen 0 und 50°C, vorzugsweise bei Raumtemperatur, durchgeführt.

b.) Abspalten eines oder zweier Schutzreste von einer Verbindung der allgemeinen Formel

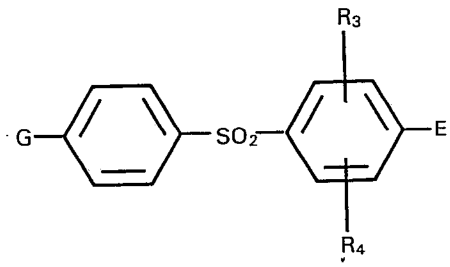

(III)

in der

$R_3$ und $R_4$ wie eingangs definiert sind,

E eine durch einen Schutzrest geschützte Amino-, Alkylamino- oder Cycloalkylaminogruppe, wobei der Alkylteil 1 bis 7 Kohlenstoffatome und der Cycloalkylteil 3 bis 7 Kohlenstoffatome enthalten kann, ohne eine $\genfrac{}{}{0pt}{}{R_1}{R_2}\!>$N-Gruppe, wobei $R_1$ und $R_2$ wie eingangs definiert sind, und

G eine gegebenenfalls durch einen Schutzrest geschützte Aminogruppe bedeuten, wobei jedoch mindestens einer der Reste E oder G eine der oben erwähnten durch einen Schutzrest geschützte Gruppe darstellen muss.

Als Schutzreste kommen die für Aminogruppen üblichen Schutzreste in Betracht, z.B. hydrolytisch abspaltbare Schutzgruppen wie die Acetyl-, Propionyl-, Benzoyl-, p-Toluolsulfonyl-, Methansulfonyl- oder Äthoxycarbonylgruppe, oder hydrogenolytisch abspaltbare Gruppen wie die Benzylgruppe.

Die Abspaltung eines Schutzrestes erfolgt zweckmässigerweise hydrolytisch, z.B. mit einer Säure wie Salzsäure, Schwefelsäure oder Phosphorsäure oder mit einer Base wie Natriumhydroxid oder Kaliumhydroxid in einem geeigneten Lösungsmittel oder Lösungsmittelgemisch wie Wasser, Wasser/Methanol, Äthanol, Wasser/Äthanol, Wasser/Isopropanol oder Wasser/Dioxan bei Temperaturen zwischen −10 und 120°C, vorzugsweise bei Temperaturen zwischen Raumtemperatur und der Siedetemperatur des Reaktionsgemisches, oder hydrogenolytisch, z.B. mit Wasserstoff in Gegenwart eines Hydrierungskatalysators wie Palladium/Kohle in einem geeigneten Lösungsmittel wie Methanol, Äthanol, Eisessig, Essigsäureäthylester, Dioxan oder Dimethylformamid bei Temperaturen zwischen 0 und 50°C, vorzugsweise bei Raumtemperatur.

c.) Zur Herstellung von Verbindungen der allgemeinen Formel I, in der $R_3$ eine Cyangruppe darstellt:

Dehydratisierung einer Verbindung der allgemeinen Formel

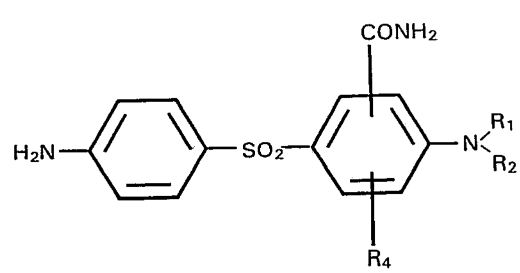

(IV)

in der

$R_1$, $R_2$ und $R_4$ wie eingangs definiert sind.

Die Dehydratisierung wird zweckmässigerweise mit einem wasserentziehenden Mittel wie Phosphorpentoxid, konzentrierte Schwefelsäure, p-Toluolsulfonsäure, Thionylchlorid, Phosphoroxychlorid, Sulfurylchlorid, Phorphorsäure oder Dicyclohexylcarbodiimid gegebenenfalls in einem Lösungsmittel wie Methylenchlorid, Pyridin, Chlorbenzol oder in einem Überschuss des verwendeten wasserentziehenden Mittels wie Thionylchlorid, Phosphoroxychlorid, Sulfurylchlorid oder Phosphorsäure bei Temperaturen zwischen 0 und 100°C, vorzugsweise jedoch bei Temperaturen zwischen 20 und 80°C, durchgeführt. Die Umsetzung kann jedoch auch ohne Lösungsmittel durchgeführt werden.

d.) Zur Herstellung von Verbindungen der allgemeinen Formel I, in der $R_3$ eine Hydroxycarbonyl-alkoxy oder Dialkylaminocarbonyl-alkoxygruppe darstellt:

Alkylierung einer Verbindung der allgemeinen Formel

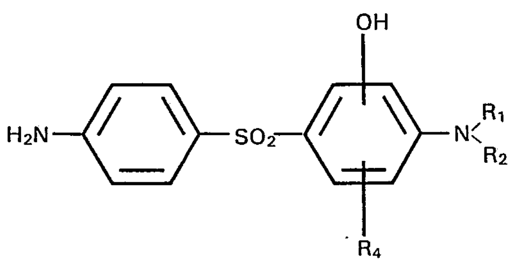

(V)

in der

$R_1$, $R_2$ und $R_4$ wie eingangs definiert sind, mit einer Verbindung der allgemeinen Formel

$$X - Alk - CO - R_5 \qquad (VI)$$

in der

Alk eine Alkylengruppe mit 1 bis 3 Kohlenstoffatomen,

$R_5$ eine Hydroxy-, Alkoxy- oder Dialkylaminogruppe, wobei der Alkylteil jeweils 1 bis 3 Kohlenstoffatome enthalten kann, und

X eine nukleophile Austrittsgruppe wie ein Chlor- oder Bromatom bedeuten, und gegebenenfalls anschliessende Hydrolyse.

Die Umsetzung wird vorzugsweise in einem Lösungsmittel wie Diäthyläther, Tetrahydrofuran, Dioxan, Methanol, Äthanol, Pyridin oder Dimethylformamid gegebenenfalls mit einer Base wie Natriumhydrid, Kaliumhydrid, Kaliumcarbonat oder Kalium-tert.butylat bei Temperaturen zwischen 0 und 75°C, vorzugsweise bei Raumtemperatur, durchgeführt.

Die gegebenenfalls anschliessende Hydrolyse wird zweckmässigerweise entweder mit einer Säure wie Salzsäure, Schwefelsäure, Phosphorsäure oder Trichloressigsäure oder mit einer Base wie Natriumhydroxid oder Kaliumhydroxid in einem geeigneten Lösungsmittel wie Wasser, Wasser/Methanol, Äthanol, Wasser/Äthanol, Wasser/Isopropanol oder Wasser/Dioxan bei Temperaturen zwischen −10 und 120°C, z.B. bei Temperaturen zwischen Raumtemperatur und der Siedetemperatur des Reaktionsgemisches, durchgeführt.

e.) Zur Herstellung von Verbindungen der allgemeinen Formel I, in der $R_3$ eine Aminocarbonyl-, Alkylaminocarbonyl-, Dialkylaminocarbonyl- oder Dialkylaminocarbonyl-alkoxygruppe darstellt:

Umsetzung einer Verbindung der allgemeinen Formel

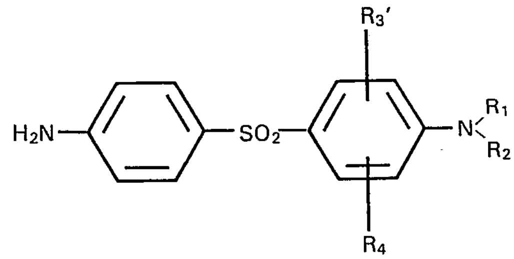

(VII)

in der

$R_1$, $R_2$ und $R_4$ wie eingangs definiert sind und

$R_3'$ eine Hydroxycarbonyl- oder Hydroxycarbonylalkoxygruppe darstellt, oder deren reaktionsfähige Derivate mit einem Amin der allgemeinen Formel

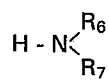

(VIII)

in der

$R_6$ ein Wasserstoffatom oder eine Alkylgruppe mit 1 bis 3 Kohlenstoffatomen und

$R_7$ ein Wasserstoffatom oder eine Alkylgruppe mit 1 bis 3 Kohlenstoffatomen bedeuten, oder dessen reaktionsfähigen Derivaten.

Die Umsetzung wird zweckmässigerweise in einem Lösungsmittel wie Methylenchlorid, Chloroform, Tetrachlorkohlenstoff, Äther, Tetrahydrofuran, Dioxan, Benzol, Toluol, Acetonitril oder Dimethylformamid, gegebenenfalls in Gegenwart eines die Säure aktivierenden Mittels oder eines wasserentziehenden Mittels, z.B. in Gegenwart von Chlorameisensäureäthylester, Thionylchlorid, Phosphortrichlorid, Phosphorpentoxid, N,N'-Dicyclohexylcarbodiimid, N,N'-Dicyclohexylcarbodiimid/N-Hydroxy-succinimid, N,N'-Carbonyldiimidazol oder N,N'-Thionyldiimidazol oder Triphenylphosphin/Tetrachlorkohlenstoff, oder eines die Aminogruppe aktivierenden Mittels, z.B. Phosphortrichlorid, und gegebenenfalls in Gegenwart einer anorganischen Base wie Natriumcarbonat oder einer tertiären organischen Base wie Triäthylamin oder Pyridin, welche gleichzeitig als Lösungsmittel dienen können, bei Temperaturen zwischen −25 und 250°C, vorzugsweise jedoch bei Temperaturen zwischen −10°C und der Siedetemperatur des verwendeten Lösungsmittels, durchgeführt. Die Umsetzung kann auch ohne Lösungsmittel durchgeführt werden, des weiteren kann während der Umsetzung entstehendes Wasser durch azeotrope Destillation, z.B. durch Erhitzen mit Toluol am Wasserabscheider, oder durch Zugabe eines Trockenmittels wie Magnesiumsulfat oder Molekularsieb abgetrennt werden.

Bei der Umsetzung kann es ferner von Vorteil sein, wenn im Reaktionsgemisch zunächst ein aktiviertes

Derivat einer Verbindung der allgemeinen Formel VII oder VIII hergestellt wird, welches anschliessend mit einer Verbindung der allgemeinen Formel VIII oder VII umgesetzt wird.

f.) Zur Herstellung von Verbindungen der allgemeinen Formel I, in der $R_3$ eine Hydroxymethyl-, Aminomethyl- oder 1-Hydroxyalkylgruppe darstellt:

Reduktion einer Verbindung der allgemeinen Formel

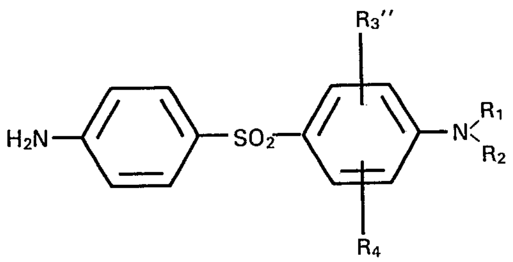

(IX)

in der

$R_1$, $R_2$ und $R_4$ wie eingangs definiert sind und

$R_3''$ eine Hydroxycarbonyl-, Alkoxycarbonyl-, Aminocarbonyl- oder Alkylcarbonylgruppe darstellt, wobei die Alkylcarbonylgruppe insgesamt 2 bis 4 Kohlenstoffatome enthalten kann.

Die Umsetzung wird vorzugsweise mit einem Metallhydrid wie Natriumborhydrid, Lithiumaluminiumhydrid oder Diboran in einem Lösungsmittel wie Wasser, Methanol, Wasser/Methanol, Diäthyläther, Tetrahydrofuran oder Dioxan bei Temperaturen zwischen 0 und 80°C, vorzugsweise bei Temperaturen zwischen der Raumtemperatur und 70°C, durchgeführt.

g.) Zur Herstellung von Verbindungen der allgemeinen Formel I, in der $R_1$ eine Alkyl- oder Cycloalkylgruppe und $R_2$ ein Wasserstoffatom darstellen:

Reduktion einer Verbindung der allgemeinen Formel

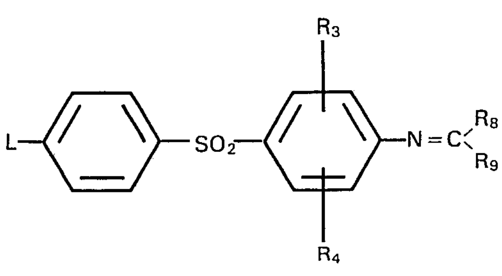

(X)

in der

$R_3$ und $R_4$ wie eingangs definiert sind,

L eine Amino- oder Nitrogruppe und

$R_8$ und $R_9$ zusammen mit dem dazwischenliegenden Kohlenstoffatom eine Alkylidengruppe mit 1 bis 7 Kohlenstoffatomen oder eine Cycloalkylidengruppe mit 3 bis 7 Kohlenstoffatomen bedeuten.

Die Reduktion wird vorzugsweise in einem geeigneten Lösungsmittel wie Methanol, Äthanol, Methanol/Wasser, Essigsäureäthylester, Tetrahydrofuran oder Dioxan mit nascierendem oder katalytisch angeregtem Wasserstoff oder mit einem Hydrid wie Diboran, Natriumborhydrid oder Lithiumaluminiumhydrid bei Temperaturen zwischen 0 und 50°C, vorzugsweise jedoch bei Raumtemperatur, durchgeführt.

Bedeutet L die Nitrogruppe so wird die Reduktion besonders vorteilhaft in einem Lösungsmittel wie Methanol oder Essigsäureäthylester mit Wasserstoff in Gegenwart eines Hydrierungskatalysators wie Platin oder Palladium/Kohle und bei einem Wasserstoffdruck von 0 bis 5 bar oder mit einem komplexen Metallhydrid wie Lithiumaluminiumhydrid oder Diboran bei Temperaturen zwischen 0 und 50°C, vorzugsweise bei Raumtemperatur, durchgeführt.

Bedeutet L in einer Verbindung der allgemeinen Formel X die Aminogruppe, so wird die Reduktion besonders vorteilhaft in einem Lösungsmittel wie Methanol, Methanol/Wasser, Tetrahydrofuran oder Dioxan mit einem komplexen Metallhydrid wie Natriumborhydrid oder Lithiumaluminiumhydrid bei Temperaturen zwischen 0 und 50°C, vorzugsweise jedoch bei Raumtemperatur, durchgeführt.

h.) Zur Herstellung von Verbindungen der allgemeinen Formel I, in der mindestens einer der Reste $R_1$ oder $R_2$ kein Wasserstoffatom darstellt und $R_3$ ein Halogenatom oder eine Alkylamino-, Dialkylamino-, Hydroxyalkyl-, Aminoalkyl-, Alkoxyalkyl-, Hydroxy-, Amino-, Trifluormethyl- oder Alkylgruppe bedeutet:

Reduktion einer Verbindung der allgemeinen Formel

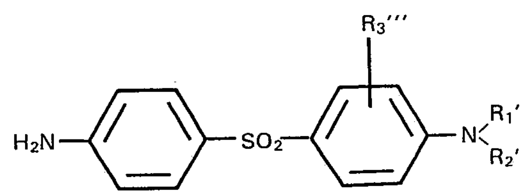

(XI)

in der

$R_1'$ und $R_2'$ die für $R_1$ und $R_2$ eingangs erwähnten Bedeutungen besitzt, wobei jedoch einer der Reste $R_1'$ oder $R_2'$ eine Alkanoylgruppe mit 1 bis 7 Kohlenstoffatomen darstellen muss, und

$R_3'''$ ein Halogenatom oder eine Alkylamino-, Dialkylamino-, Hydroxyalkyl-, Aminoalkyl-, Alkoxyalkyl-, Hydroxy-, Amino-, Trifluormethyl- oder Alkylgruppe, wobei der Alkylteil jeweils 1 bis 3 Kohlenstoffatome enthalten kann, bedeutet.

Die Reduktion wird vorzugsweise in einem Lösungsmittel wie Methanol, Äthanol, Äther, Tetrahydrofuran oder Dioxan mit einem geeigneten Reduktionsmittel wie einem Metallhydrid, z.B. Lithiumaluminiumhydrid, Natrium-bis-(methoxyäthoxy)-aluminium-dihydrid, mit Natriumborhydrid in Gegenwart einer Lewis-Säure wie Aluminiumchlorid, Titan(IV)-chlorid oder Kobalt(II)chlorid, mit Natriumborhydrid in Gegenwart von Trifluoressigsäure, Essigsäure oder Pyridin, mit Natriumborhydrid/Triäthyloxoniumtetrafluorborat, mit Diboran oder Boran/Dimethylsulfid, bei Temperaturen zwischen −10 und 120°C, vorzugsweise zwischen 0°C und der Siedetemperatur des verwendeten Lösungsmittels, durchgeführt.

Erhält man erfindungsgemäss eine Verbindung der allgemeinen Formel I, in der $R_3$ eine Alkoxycarbonyl- oder Alkoxycarbonyl-alkoxygruppe darstellt, so kann diese mittels Hydrolyse in eine entsprechende Verbindung der allgemeinen Formel I, in der $R_3$ eine Hydroxycarbonyl- oder Hydroxycarbonyl-alkoxygruppe darstellt, übergeführt werden oder

eine Verbindung der allgemeinen Formel I, in der $R_3$ und/oder $R_4$ ein Chlor- oder Bromatom darstellen, so kann diese mittels Hydrolyse oder Alkoholyse in eine entsprechende Verbindung der allgemeinen Formel I, in der $R_3$ in 2-Stellung eine Hydroxy- oder Alkoxygruppe und $R_4$ ein Chlor- oder Bromatom darstellen, übergeführt werden.

Diese Hydrolyse wird zweckmässigerweise entweder mit einer Säure wie Salzsäure oder Schwefelsäure, oder mit einer Base wie Natriumhydroxid oder Kaliumhydroxid in einem geeigneten Lösungsmittel wie Wasser, Wasser/Methanol, Äthanol, Wasser/Äthanol, Wasser/Isopropanol oder Wasser/Dioxan bei Temperaturen zwischen −10 und 120°C, z.B. bei Temperaturen zwischen Raumtemperatur und der Siedetemperatur des Reaktionsgemisches, durchgeführt.

Die Alkoholyse wird zweckmässigerweise in einem entsprechenden Alkohol als Lösungsmittel wie Methanol, Äthanol oder Propanol, gegebenenfalls in einem Druckgefäss vorzugsweise mit einer Base wie Natriumhydroxid oder Kaliumhydroxid bei Temperaturen zwischen 20 und 200°C, vorzugsweise bei Temperaturen zwischen 50 und 180°C, durchgeführt.

Die als Ausgangsstoffe verwendeten Verbindungen der allgemeinen Formeln II bis XI sind literaturbekannt, bzw. man erhält diese nach literaturbekannten Verfahren.

So erhält man beispielsweise eine Verbindung der allgemeinen Formel II oder III durch Umsetzung eines Alkalisalzes einer entsprechenden Acylamino-phenylsulfinsäure mit einem entsprechenden Halogennitrobenzol. Eine gegebenenfalls nach Abspaltung eines als Schutzrest verwendeten Acylrestes so erhaltene Verbindung der allgemeinen Formel

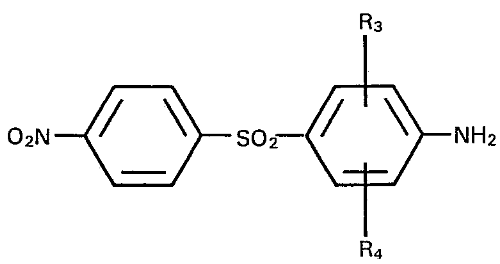

(XII)

in der

$R_3$ und $R_4$ wie eingangs definiert sind, kann anschliessend mittels reduktiver Aminierung in eine Verbindung der allgemeinen Formel II bzw. nach Tosylierung, anschliessender Alkylierung und Reduktion der Nitrogruppe und gegebenenfalls gleichzeitiger Acylierung in eine Verbindung der allgemeinen Formel III oder XI übergeführt werden.

Ferner kann eine nach literaturbekannten Verfahren hergestellte Verbindung der allgemeinen Formel

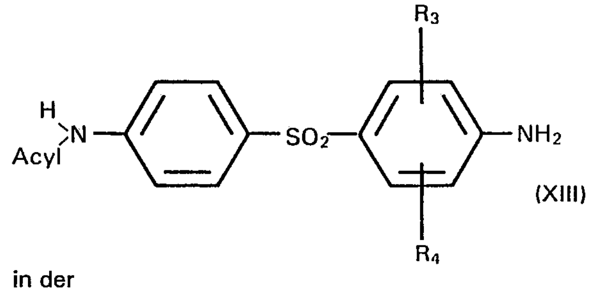

(XIII)

in der

$R_3$ und $R_4$ wie eingangs definiert sind und

Acyl eine organische Acylgruppe darstellt, mittels reduzierender Aminierung oder durch Reduktion eines nach der Umsetzung mit einer entsprechenden Carbonylverbindung erhaltenen Schiff'schen Base in eine Verbindung der allgemeinen Formel III übergeführt werden, in der G einen Acylaminorest und

E eine $R_1 R_2$>N-Gruppe bedeuten.

Des weiteren kann eine Verbindung der allgemeinen Formel

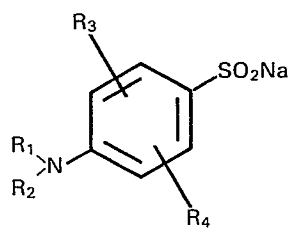

(XIV)

in der

$R_1$ bis $R_4$ wie eingangs definiert sind und

Na ein Natriumion darstellt, mit einem 4-Halogen-nitrobenzol zu einem entsprechenden Diphenylsulfon der allgemeinen Formel III umgesetzt werden.

Eine Cycloalkylaminoverbindung der allgemeinen Formel II erhält man jedoch vorzugsweise durch reduktive Aminierung einer entsprechenden Aminoverbindung mit einem Cycloalkanon in Gegenwart von Natrium-cyanoborhydrid oder durch Reduktion der entsprechenden Schiff'schen Base mit einem komplexen Metallhydrid.

Eine als Ausgangsstoff verwendete Verbindung der allgemeinen Formel X erhält man durch Umsetzung einer entsprechenden Verbindung der allgemeinen Formel XII mit einem entsprechenden Carbonylderivat gegebenenfalls in Gegenwart von Titan(IV)-chlorid und gegebenenfalls anschliessende Reduktion der Nitrogruppe.

Wie bereits eingangs erwähnt, weisen die neuen Verbindungen der allgemeinen Formel I sowie deren Säureadditionssalze mit physiologisch verträglichen anorganischen oder organischen Säuren eine Hemmwirkung auf das Wachstum von Bakterien und Parasiten wie Plasmodien und Mykobakterien aufgrund ihrer Hemmwirkung auf die 7,8-Dihydropteroinsäure-Synthetase auf.

Beispielsweise wurden die Verbindungen

A = 4-Äthylamino-4'-amino-2-chlor-diphenylsulfon,

B = 4'-Amino-2-chlor-4-isopropylamino-diphenylsulfon,
C = 4-Äthylamino-4'-amino-2-methyl-diphenylsulfon,
D = 4-Äthylamino-4'-amino-2-trifluormethyl-diphenylsulfon,
E = 4,4'-Diamino-2-hydroxymethyl-diphenylsulfon,
F = 4,4'-Diamino-2-(1-hydroxyäthyl)-diphenylsulfon,
G = 4,4'-Diamino-2-(N,N-dimethylamino)-diphenylsulfon
H = 4,4'-Diamino-2-(N-methylamino)-diphenylsulfon,
I = 4,4'-Diamino-2-cyano-diphenylsulfon,
K = 4,4'-Diamino-2-methylcarbonyl-diphenylsulfon,
L = 4'-Amino-2-methyl-4-methylamino-diphenylsulfon und
M = 4'-Amino-2-methyl-4-propylamino-diphenylsulfon

auf ihre biologische Aktivität im zellfreien Enzymextrakt von Plasmodium berghei wie folgt untersucht:

*1. Präparation des Enzymextraktes*

Plasmodien werden entsprechend der folgenden Literaturstelle [Heidrich, H.-G. et al., Z. Parasitenkd. 59:151(1979)] aus dem mit Plasmodium berghei infiziertem Mäuseblut isoliert. Der Aufschluss der Plasmodien erfolgt durch Ultraschall. Die Anreicherung von Proteinen mit 7,8-Dihydropteroinsäure-Synthetase-Aktivität gelingt mit Hilfe der Gelfiltration.

*2. Biologisches Testsystem*

Die Hemmwirkung auf die 7,8-Dihydropteroinsäure-Synthese der Plasmodien wird wie folgt ermittelt:

Unter optimalen Reaktionsbedingungen, d.h. $V_{max}$ für die zu hemmende Reaktion, wird die Hemmstoff-Konzentration der neuen Verbindungen ermittelt, die zu 50% Reduktion in der Enzymsynthese-Leistung führt. Dazu wird die von dem Enzymextraxt synthetisierte Menge an 7,8-Dihydropteroinsäure mit Hilfe der High-Performance-Liquid-Chromatographie (HPLC) unter Verwendung eines UV-Detektors nach 4 Stunden Inkubationszeit ermittelt. Es wurde sichergestellt, dass während dieses Zeitraumes die Synthese der 7,8-Dihydropteroinsäure linear verläuft.

Die nach diesem Verfahren ermittelten $i_{50}$-Werte für eine Auswahl der beanspruchten Verbindungen und die der Vergleichspräparate Bis(4-amino-phenyl)sulfon (Dapsone®, DDS) und $N^1$-(5,6-Dimethoxy-4-pyrimidinyl)-sulfanilamid (Fanasil®) sind in der nachfolgenden Tabelle wiedergegeben:

| Substanz | $i_{50}$ [µM] |
|---|---|
| A | 2.10 |
| B | 2.67 |
| C | 2.62 |
| D | 5.86 |
| E | 4.90 |
| F | 10.40 |
| G | 12.90 |
| H | 4.90 |
| I | 12.48 |
| K | 12.90 |
| L | 2.94 |
| M | 2.44 |
| Dapson® | 12.41 |
| Fanasil® | 200 |

Ferner weisen die neuen Verbindungen im Gegensatz zu Dapson® eine gute Verträglichkeit auf, insbesondere eine sehr geringe Methämoglobinbildung. Beispielsweise konnte an der Katze bei einer Applikation der Substanzen I und M in einer Dosis von 200 mg/kg p.o. keine Methämoglobinbildung beobachtet werden.

Aufgrund ihrer biologischen Eigenschaften eignen sich die neuen Verbindungen sowie ihre physiologisch verträglichen Säureadditionssalze zur Behandlung bakterieller Erkrankungen, der Malaria und der Lepra.

Bei der Monotherapie beträgt hierbei am Erwachsenen die Einzeldosis 100 bis 300 mg, vorzugsweise jedoch 100 bis 200 mg, 1- bis 2mal täglich; bei der Kombinationstherapie mit einem Dihydrofolsäure-Reduktase-Hemmer beträgt am Erwachsenen die Einzeldosis 50 bis 200 mg + 5 bis 30 mg Pyrimethamin, vorzugsweise jedoch 50 bis 150 mg + 6,25 bis 25 mg Pyrimethamin.

Hierzu lassen sich die neuen Verbindungen sowie ihre physiologisch verträglichen Säureadditionssalze gegebenenfalls in Kombination mit einen Dihydrofolsäure-Reduktase-Hemmer wie Pyrimethamin, Trimethoprim oder einem Trimethoprim-Derivat in die üblichen galenischen Zubereitungen wie Dragées, Tabletten, Kapseln oder Suspensionen einarbeiten.

Die nachfolgenden Beispiele sollen die Erfindung näher erläutern:

*Beispiel 1*

*3-Cyano-4,4'-diamino-diphenylsulfon*

*a) 3-Cyano-4,4'-dinitro-diphenylsulfid*

a) 10 g (0,064 Mol) 4-Nitrothiophenol werden durch Rühren in einer Lösung von 2,8 g Natriumhydroxid in 10 ml Wasser und 100 ml Äthanol gelöst. Anschliessend werden 11,8 g (0,064 Mol) 5-Chlor-2-nitrobenzonitril zugegeben, um dann die resultierende Lösung unter Rühren 1 Stunde am Rückfluss zu erhitzen, wobei eine gelbe Substanz ausfällt. Nach Erkalten der Suspension wird das angefallene Produkt abgesaugt, mit Äthanol gewaschen und dann getrocknet. Dieses Proprodukt wird aus Methanol umkristallisiert.

Gelbe Kristalle vom Schmelzpunkt: 123-126°C.

*b) 3-Cyano-4,4'-dinitro-diphenylsulfon*

10 g (0,033 Mol) 3-Cyano-4,4'-dinitro-diphenylsulfid werden durch Erwärmen in 150 ml Eisessig gelöst. Diese Lösung wird unter Rühren bei Raumtemperatur tropfenweise mit 20 ml Perhydrol versetzt. Nach beendeter Zugabe wird noch $1^1/_2$ Stunden am Rückfluss erhitzt, wobei eine weisse Substanz ausfällt. Nach Erkalten der Suspension wird die Substanz abgesaugt, mit Äthanol und Wasser gewaschen und dann getrocknet.

Gelbe Kristalle vom Schmelzpunkt: 188-190°C.

*c) 3-Cyano-4,4'-diamino-diphenylsulfon*

2 g (0,006 Mol) 3-Cyano-4,4'-dinitro-diphenylsulfon werden unter Rühren bei 10-20°C portionsweise in einer Mischung von 8,1 g $SnCl_2 \times 2H_2O$ und 15 ml konz. Salzsäure eingetragen. Nach beendeter Zugabe wird noch 4 Stunden bei Raumtemperatur nachgerührt, um dann die Mischung unter starker Kühlung in 50 ml 10 n Natronlauge einzurühren. Das ausgefallene Produkt wird abgesaugt und getrocknet. Das Rohprodukt wird säulenchromatographisch gereinigt. Hierzu wird die Substanz in Essigester gelöst und über 200 g Kieselgel (0,063-0,2 mm) chromatographiert, wobei Essigester/Methylenchlorid 1:1 als Elutionsmittel dient. Die entsprechenden Fraktionen werden vereinigt und eingeengt.

Farblose Kristalle vom Schmelzpunkt: 240 bis 242°C.

*Beispiel 2*

*4,4'-Diamino-2,6-dibrom-diphenylsulfon*

*a) 3,4,5-Tribromnitrobenzol*

In einer Lösung von 12 g Natriumnitrit in 85 ml konzentrierter Schwefelsäure lässt man bei 20°C unter Rühren und Kühlen mit Eis eine Lösung von 47,2 g 2,6-Dibrom-4-nitro-anilin in 1,6 l Essigsäure langsam einlaufen. Anschliessend rührt man während 20 Minuten bei Raumtemperatur. Die so bereitete Lösung gibt man nun unter Rühren und Kühlen mit Eis langsam zu einer Lösung von 12,8 g Kupfer-(I)-bromid in 40 ml Bromwasserstoffsäure (63%ig). Nach beendeter Zugabe wird weitere 30 Minuten gerührt. Man verdünnt mit Wasser, filtriert den Niederschlag ab und wäscht mit Wasser. Dieses Rohprodukt wird aus Methanol umkristallisiert.

*b) 4'-Acetamino-2,6-dibrom-4-nitro-diphenylsulfid*

2,9 g Natrium werden in 520 ml absolutem Äthanol gelöst. In dieser so zubereiteten Natriumalkoholat-Lösung löst man 22 g 4-Acetamino-thiophenol, gibt anschliessend 44,5 g 3,4,5-Tribrom-nitrobenzol hinzu und erhitzt dann während 5 Stunden auf Rückflusstemperatur. Nach dem Abkühlen giesst man auf Wasser, extrahiert erschöpfend mit Essigester, wäscht die vereinigten Essigester-Extrakte mit Wasser, trocknet über Natriumsulfat und engt auf ein kleines Volumen ein. Das ausgeschiedene Material wird abgesaugt und aus Methanol umkristallisiert.

Farblose Kristalle vom Schmelzpunkt: 208 bis 209°C.

*c) 2,6-Dibrom-4,4'-dinitro-diphenylsulfon*

5 g 4'-Acetamido-2,6-dibrom-4-nitro-diphenylsulfid werden durch Erwärmen in 40 ml Eisessig gelöst. Diese Lösung wird unter Rühren bei Raumtemperatur tropfenweise mit 20 ml Perhydrol versetzt. Nach beendeter Zugabe wird noch 2 Stunden lang auf Rückflusstemperatur erhitzt. Beim Abkühlen scheiden sich gelbe Kristalle ab, die abgesaugt und zuerst mit Wasser und dann mit Methanol gewaschen werden.

*d) 4,4'-Diamino-2,6-dibrom-diphenylsulfon*

17 g Zinn(II)chlorid-dihydrat werden in 17 ml konzentrierter Salzsäure gelöst. Unter Rühren trägt man in diese Lösung spatelweise 3,96 g 2,6-Dibrom-4,4'--dinitro-diphenylsulfon ein, wobei die Temperatur auf etwa 50°C ansteigt. Man rührt weitere 2 Stunden und lässt während 16 Stunden bei Raumtemperatur stehen. Anschliessend giesst man unter Rühren und Kühlen mit Eis langsam in 40 ml einer 10 n Natronlauge ein. Der Niederschlag wird abfiltriert und gut mit Wasser und anschliessend mit Isopropanol gewaschen.

Farblose Kristalle vom Schmelzpunkt: 168 bis 170°C (Zers.).

*Beispiel 3*

*4,4'-Diamino-2-hydroxy-6-methoxy-diphenylsulfon*

1 g 4,4'-Diamino-2,6-dibrom-diphenylsulfon (Beispiel 2) werden zu 1,12 g Kaliumhydroxid in 0,15 ml Wasser und 25 ml Methanol gegeben. Diese Mischung wird unter Druck während 14 Stunden auf 150°C erhitzt. Anschliessend wird mit Wasser verdünt, vom Unlöslichen abfiltriert und im Vakuum zur Trockne eingeengt. Der feste Rückstand wird in wenig Wasser aufgenommen. Die wässrige Lösung wird mit 2n Salzsäure bis pH 5 angesäuert und der ausgeschiedene farblose Niederschlag abgesaugt und mit Wasser gewaschen und getrocknet.

Schmelzpunkt: 127°C (Zers.).

*Beispiel 4*

*2-Brom-4,4'-diamino-6-hydroxy-diphenylsulfon*

1 g 4,4'-Diamino-2,6-dibrom-diphenylsulfon (Beispiel 2) werden zu einer Lösung von 1,12 g Kaliumhydroxid in 0,15 ml Wasser und 50 ml tert.-Butanol gegeben. Diese Mischung wird unter Druck während 10 Stunden auf 150°C erhitzt. Anschliessend wird mit Wasser verdünnt, vom Unlöslichen abfiltriert und im Vakuum zur Trockne eingeengt. Der feste Rückstand wird in wenig Wasser aufgenommen. Die wässrige Lösung wird mit 2n Salzsäure bis pH 5 angesäuert und der ausgeschiedene farblose Niederschlag abgesaugt und mit Wasser gewaschen und getrocknet.

IR-Spektrum (KBr):

| | |
|---|---|
| 3000 - 3600 $cm^{-1}$ | OH assoz. |
| 3360, 3470 $cm^{-1}$ | $NH_2$ frei |
| 3210 $cm^{-1}$ | $NH_2$ assoz. |
| 2840 $cm^{-1}$ | $OCH_3$ |
| 1140, 1320 $cm^{-1}$ | $SO_2$ |

*Beispiel 5*

*4-Äthylamino-4'-amino-2-methyl-diphenylsulfon*

*a) 4'-Acetamino-2-methyl-4-tosylamino-diphenylsulfid*

2,7 g 4'-Acetamino-4-amino-2-methyl-diphenylsulfid [J. org. Chem. 15, 400 (1950)] werden in 7 ml trockenem Pyridin gelöst. Zu dieser Lösung gibt man bei Raumtemperatur 2,85 g p-Toluolsulfonsäurechlorid und lässt 2 Tage bei Raumtemperatur stehen. Anschliessend erhitzt man zur vollständigen Umsetzung während 6 Stunden auf 90°C und verdünnt nach Abkühlen mit etwa 50 ml Wasser. Der ausgeschiedene Festkörper wird abgesaugt und sorgfältig mit Wasser und anschliessend mit Petroläther gewaschen und getrocknet.

Schmelzpunkt: 130-134°C.

*b) 4'-Acetamino-4-(N-äthyl-N-tosylamino)-2-methyl-diphenylsulfid*

3,5 g 4'-Acetamino-2-methyl-4-tosylamino-diphenylsulfid, 2 g Äthyljodid und 1,5 g trockenes Kaliumcarbonat werden in 50 ml Dimethylformamid unter Rühren während 17 Stunden auf 95°C erhitzt. Nach Abkühlen giesst man auf Wasser. Der ausgeschiedene Festkörper wird abgesaugt und gut mit Wasser und Petroläther gewaschen und getrocknet.

IR-Spektrum (Methylenchlorid):

| | |
|---|---|
| 3420 $cm^{-1}$ | NH |
| 1695 $cm^{-1}$ | Amid |
| 1505 $cm^{-1}$ | Sulfonamid |

*c) 4'-Acetamino-4-(N-äthyl-tosylamino)-2-methyl-diphenylsulfon*

2,25 g 4'-Acetamino-4-(N-äthyl-tosylamino)-2-methyl-diphenylsulfid werden in 20 ml Essigsäure und 6 ml Perhydrol während 3 Stunden auf 90°C erhitzt. Nach Abkühlen wird mit Wasser verdünnt, der ausgeschiedene Festkörper sorgfältig mit Wasser und Petroläther gewaschen und getrocknet. Dieses Rohprodukt wird über Kieselgel mit einem Methylenchlorid-Methanol-Gemisch (25:1) chromatographiert.

Hellgelbe schaumige Substanz.

IR-Spektrum (KBr):

| | |
|---|---|
| 3420 $cm^{-1}$ | NH |
| 1680 $cm^{-1}$ | Amid |
| 1500 $cm^{-1}$ | Sulfonamid |
| 1315, 1150 $cm^{-1}$ | Sulfon |

*e) 4-Äthylamino-4'-amino-2-methyl-diphenylsulfon*

1,05 g 4'-Acetamino-4-(N-äthyl-tosylamino)-2-methyl-diphenylsulfon werden in 6 ml konzentrierter Schwefelsäure gelöst. Diese Lösung lässt man während 4 Stunden bei Raumtemperatur stehen, versetzt anschliessend mit Eis und neutralisiert mit Ammoniak. Der ausgefallene Festkörper wird abgesaugt. Das Rohprodukt, bestehend aus 4'-Acetamino-4-äthylamino-2-methyl-diphenylsulfon wird während einer Stunde in einer Mischung aus konzentrierter Salzsäure/Wasser = 1/1 auf Siedetemperatur erhitzt. Nach dem Abkühlen wird auf Wasser gegossen und mit Ammoniak neutralisiert. Der ausgeschiedene Festkörper wird mit Wasser gewaschen und getrocknet.

Schmelzpunkt: 166-167°C.

*Beispiel 6*

*4'-Amino-2-methyl-4-methylamino-diphenylsulfon*

3,5 g 4'-Acetamino-2-methyl-4-(N-methyl-tosyl-amino)-diphenylsulfon, hergestellt analog Beispiel 5, werden zusammen mit 3,5 g Phenol in 120 ml 48%iger Bromwasserstoffsäure während 1,5 Stunden auf Rückflusstemperatur erhitzt. Nach Abkühlen giesst man auf Eis, neutralisiert mit Natriumhydroxid, extrahiert mit Methylenchlorid, trocknet über Natriumsulfat und engt im Vakuum zur Trockne ein. Der feste Rückstand wird in absolutem Äthanol gelöst. Diese Lösung wird mit äthanolischer Salzsäure angesäuert und dann mit so viel Äther versetzt, dass Kristallisation eintritt. Die Kristalle werden abgesaugt, mit Äthanol/Äther gewaschen und getrocknet.

Dihydrochlorid: Farblose Kristalle vom Schmelzpunkt: 232-235°C.

*Beispiel 7*

*4'-Amino-2-methyl-4-n-propylamino-diphenylsulfon*

Hergestellt aus 4'-Acetamino-2-methyl-4-(N-n-propyl-tosylamino)-diphenylsulfon mit Phenol und Bromwasserstoffsäure analog Beispiel 6.

Farblose Kristalle vom Schmelzpunkt: 170 bis 173°C.

*Beispiel 8*

*4-Äthylamino-4'-amino-2-chlor-diphenylsulfon*

*a) 4'-Acetamino-2-chlor-4-p-tosylamino-diphenylsulfon*

190 mg p-Tosylchlorid werden in 5 ml wasserfreiem Pyridin gelöst und 300 mg 4'-Acetamino-4-amino-2-chlor-diphenylsulfon [Synthese nach J. Med. Chem. 14:1166 (1971)] zugegeben. Der Ansatz wird unter Rühren 3 Stunden bei Raumtemperatur und eine Stunde bei 50°C gehalten. Nach dem Abkühlen wird auf Eis/HCl gegeben und der Niederschlag abgesaugt.

Umkristallisieren aus MeOH/$H_2O$, farblose Kristalle.

*b) 4-Äthylamino-4'-amino-2-chlor-diphenylsulfon*

70 mg 4'-Acetamino-2-chlor-4-p-tosylamino-diphenylsulfon und 25 mg Kaliumcarbonat werden in 5 ml DMF suspendiert. Nach Zusatz von 200 µl Äthyljodid wird 24 Stunden auf 90°C (Bad) erwärmt. Nach dem Abkühlen wird auf Eis gegeben und der Niederschlag abgesaugt. Der trockene Rückstand wird unter Rühren in 0,5 ml konz. $H_2SO_4$ gelöst. Nach einer Stunde wird auf Eis gegossen und der pH-Wert mit Ammoniak auf pH 4-6 gebracht. Der ausgefallene Niederschlag wird abgesaugt, mit 10

ml 20%iger Salzsäure versetzt und eine Stunde unter Rückfluss erhitzt. Nach dem Abkühlen wird mit Ammoniak neutralisiert und der Niederschlag abgesaugt.

Umkristallisieren aus MeOH/$H_2O$.
Schmelzpunkt: 173-175°C.

*Beispiel 9*

*4'-Amino-2-chlor-4-isopropylamino-diphenylsulfon*

300 mg 4'-Amino-2-chlor-4-p-tosylamino-diphenylsulfon, 100 mg Kaliumcarbonat, 500 µl Isopropylbromid und 10 ml DMF werden 72 Stunden auf 90°C (Bad) erwärmt. Nach dem Abkühlen wird auf Eis gegeben und der Niederschlag abgesaugt. Der trockene Rückstand wird unter Rühren in 0,5 ml konz. $H_2SO_4$ gelöst. Nach einer Stunde wird auf Eis gegeben und der pH-Wert mit $NH_3$ auf 4-6 gebracht. Der ausgefallene Niederschlag wird abgesaugt, mit 10 ml 20%iger Salzsäure versetzt und eine Stunde unter Rückfluss erhitzt. Nach dem Abkühlen wird mit Ammoniak neutralisiert und der Niederschlag abgesaugt.

Umkristallisieren aus Methanol. Farbloser Festkörper.

| | | | |
|---|---|---|---|
| NMR (DMSO) | 1.08 - 1.15 | 6H | Dublett $CH(CH_3)_2$ |
| 90 MHZ | 3.4 - 3.7 | 1H | Multiplett $\underline{CH}(CH_3)_2$ |
| | 6.04 | 2H | Singulett $\underline{NH_2}$ |
| | 6.5 - 6.7 | 5H | Multiplett NH, aromatische H |
| | 7.4 - 7.5 | 2H | Dublett, aromatische H |
| | 7.75-7.85 | 1H | Dublette, aromatisches H |

*Beispiel 10*

*2-Carboxymethyloxy-4,4'-diamino-diphenylsulfon*

*a) 2-Äthoxycarbonylmethyloxy-4,4'-diamino-diphenylsulfon*

7,9 g (0,03 Mol) 4,4'-Diamino-2-hydroxydiphenylsulfon [J. Scientific and Industrial Research 17B, 192 (1958)] werden in 160 ml Tetrahydrofuran gelöst; diese Lösung wird unter Stickstoffatmosphäre und Rühren bei Raumtemperatur mit 1,3 g (0,03 Mol) Natriumhydrid versetzt; dabei beginnt das Gemisch zu schäumen, gegen Ende der Zugabe fällt ein dicker Kristallbrei aus. Durch Zugabe von 250 ml Dimethylformamid erhält man eine klare Lösung. Man tropft 3,3 ml (0,03 Mol) Bromessigsäureäthylester zu, rührt das Gemisch 3,5 Stunden lang bei Raumtemperatur und engt dann i.V. zur Trockne ein. Der Rückstand wird mit einem Gemisch aus Wasser und Methylenchlorid verrührt, wobei man ein festes kristallines Material erhält, das vom Lösungsmittelgemisch abgesaugt, erneut mit Methylenchlorid/Wasser verrührt, wieder abgesaugt und getrocknet wird. Das so erhaltene Produkt hat einen Schmelzpunkt von 178 bis 183°C.

*b) 2-Carboxymethyloxy-4,4'-diamino-diphenylsulfon*

2,8 g (0,008 Mol) 2-Äthoxycarbonylmethyloxy-4,4'-diamino-diphenylsulfon werden in einem Gemisch aus 280 ml Äthanol, 2,8 g (0,007 Mol) Natriumhydroxid und 56 ml Wasser eine halbe Stunde zum Sieden erhitzt. Man lässt abkühlen, säuert mit konz. Salzsäure bis zu einem pH-Wert von 1 an, engt i.V. zur Trockne ein, kocht den Rückstand kurz mit Alkohol auf, filtriert von unlöslichen Bestandteilen ab, engt das Filtrat i.V. zur Trockne ein und reinigt den Rückstand durch Chromatographie an Kieselgel, Elutionsmittel Methylenchlorid/Methanol (1:1). Das so erhaltene Produkt wird aus Äthanol umkristallisiert und hat dann einen Schmelzpunkt von 255 bis 260°C (Zers.).

*Beispiel 11*

*4,4'-Diamino-2-dimethylamino-carbonyl-methyloxy-diphenylsulfon*

3,4 g (0,0097 Mol) 2-Äthoxycarbonylmethyloxy-4,4'-diamino-diphenyl-sulfon werden in einem Gemisch aus 68 ml wässriger 40%iger Dimethylaminlösung und 100 ml Tetrahydrofuran gelöst. Man lässt diese Lösung 3 Stunden bei Raumtemperatur stehen, dampft dann i.V. zur Trockne ein, verreibt den erhaltenen Rückstand mit 100 ml Wasser, saugt das feste Material ab und kristallisiert aus Äthanol um. Das so erhaltene Produkt hat einen Schmelzpunkt von 238-244°C (Zers.).

*Beispiel 12*

*4,4'-Diamino-2-methoxymethyl-diphenylsulfon*

*a) 2-Hydroxymethyl-4-nitro-chlorbenzol*

43,2 g (0,215 Mol) 2-Carboxy-4-nitro-chlorbenzol werden in 500 ml absolutem Tetrahydrofuran gelöst, dazu gibt man 30 ml Triäthylamin (0,215 Mol). Nun kühlt man die obige Mischung auf −10°C ab und tropft bei dieser Temperatur 22 ml Carbäthoxychlorid (0,229 Mol) dazu. Anschliessend rührt man eine Stunde bei −10 bis −5°C nach. Nun filtriert man das gebildete Triäthylamin-hydrochlorid ab. Die Tetrahydrofuranlösung tropft man nun während 10 Minuten bei 0°C zu einer Lösung von 30 g Natriumborhydrid (0,789 Mol) in 100 ml Wasser. Man lässt innerhalb einer Stunde die Reaktionsmischung unter Rühren auf Raumtemperatur kommen. Man zersetzt die erhaltene orangerote Lösung unter Eiskühlung mit 2n Salzsäure und extrahiert mit Äther. Der Ätherextrakt wird mit Wasser gewaschen. Nach Abdampfen des Äthers verbleibt ein Öl, welches nach einiger Zeit kristallisiert.

IR-Spektrum (Methylenchlorid):
3610 $cm^{-1}$ OH
1375 $cm^{-1}$ $NO_2$

*b) 2-Methoxymethyl-4-nitro-chlorbenzol*

18,7 g (0,1 Mol) 2-Hydroxymethyl-4-nitro-chlorbenzol werden in 500 ml absolutem Tetrahydrofuran gelöst und auf 0°C gekühlt. Mit 4,8 g (0,1 Mol) Natriumhydrid (55%ig in Öl) wird das Natriumalkoholat der obigen Verbindung hergestellt. Nach einer Stunde Reaktionszeit gibt man bei 0°C tropfenweise unter Rühren 14,2 g (Methyljodid (0,1 Mol) zu. Man hält

die Reaktionsmischung weitere 3 Stunden unter Rühren bei 0°C. Anschliessend rührt man ohne Kältebad bis zum Erreichen der Raumtemperatur weiter. Nun wird das Lösungsmittel abgedampft und der Rückstand in Essigester aufgenommen, die Essigesterphase mit Wasser gewaschen, getrocknet und im Vakuum zur Trockne eingedampft. Das verbleibende Öl wird aus Essigester kristallisiert.

| | | | |
|---|---|---|---|
| NMR ($CDCl_3$/$CD_3OD$): | 3,6 ppm | 3H | Singulett |
| | 4,6 ppm | 2H | Singulett |
| 80 MHz | 7,5-9,75 ppm | 3H | Multiplett |

*c) 4'-Acetylamino-4-nitro-2-methoxymethyl-diphenylsulfon*

6,5 g (0,0327 Mol) 4-Acetylanilidosulfinsäure werden in 50 ml Äthanol suspendiert und mit 1,5 g (0,033 Mol) Natriumhydrid (55%ig in Öl) in Portionen unter Rühren versetzt. Anschliessend fällt man das so erhaltene Natriumsalz der obigen Säure mit Äther aus und trocknet es. 6,6 g (0,03 Mol) 4-Acetylanilidonatriumsulfinat werden mit 6,1 g (0,03 Mol) 2-Methoxymethyl-4-nitro-chlorbenzol in 25 ml Dimethylformamid während 5 Stunden am Rückfluss erhitzt. Anschliessend wird die Reaktionsmischung auf Eiswasser gegossen und mit Essigester extrahiert. Man wäscht den Essigesterextrakt 2 × mit Wasser, trocknet über Natriumsulfat und dampft den Essigester im Vakuum ab. Man wäscht den festen Rückstand mit i-Propanol und Petroläther.

IR-Spektrum ($CH_2Cl_2$):

| | |
|---|---|
| 3420 $cm^{-1}$ | NH |
| 1710 $cm^{-1}$ | CO |
| 1350 + 1530 $cm^{-1}$ | $NO_2$ |
| 1150 + 1320 $cm^{-1}$ | $SO_2$ |

*d) 4'-Acetylamino-4-amino-2-methoxymethyl--diphenylsulfon*

3,8 g (0,0104 Mol) 4'-Acetylamino-4-nitro-2--methoxymethyl-diphenyl-sulfon werden in Portionen innerhalb von 15 Minuten zu einer siedenden Mischung von 3,4 g (0,061 Mol) Eisenpulver in 25 ml Alkohol, 6 ml Wasser und 0,05 ml 36%iger Salzsäure gegeben. Anschliessend erhitzt man während 2 Stunden auf Rückflusstemperatur. Man verdünnt die Reaktionsmischung nach Abkühlen mit Methanol, filtriert über Celite und verdampft das Methanol. Den Rückstand nimmt man in Wasser auf und extrahiert mit Essigester. Nun verdampft man den Essigester und es verbleibt ein amorphes, festes Produkt.

IR-Spektrum (KBr):

| | |
|---|---|
| 1680 $cm^{-1}$ | Amid I |
| 1525 $cm^{-1}$ | Amid II |
| 1140, 1320 $cm^{-1}$ | $SO_2$ |

*e) 4,4'-Diamino-2-methodymethyl-diphenylsulfon*

3,5 g (0,01 Mol) 4'-Acetylamino-4-amino-2-methoxymethyl-diphenylsulfon werden in 3n Salzsäure während 15 Minuten auf Siedetemperatur erhitzt. Die Reaktionslösung wird nach Abkühlen mit Wasser verdünnt und filtriert. Das wässrige Filtrat wird mit verdünntem wässrigen Ammoniak auf pH 8-9 gebracht. Der dabei ausfallende Niederschlag wird abfiltriert und mit Wasser gewaschen.

IR-Spektrum (KBr):

| | |
|---|---|
| 1130 $cm^{-1}$ | $SO_2$ |
| 1280 $cm^{-1}$ | $SO_2$ |

*Beispiel 13*

*4,4'-Diamino-2-methylcarbonyl-diphenylsulfon*

Hergestellt aus 4'-Acetamino-4-amino-2-methylcarbonyl-diphenylsulfon (Darstellung analog Beispiel 12c und 12d) mit Salzsäure analog Beispiel 12e.

IR-Spektrum ($CH_2Cl_2$):

| | |
|---|---|
| 3485, 3390 $cm^{-1}$ | $NH_2$ |
| 1695 $cm^{-1}$ | Keton |
| 1290, 1140 $cm^{-1}$ | $SO_2$ |

*Beispiel 14*

*4,4'-Diamino-2-(1-hydroxyäthyl)-diphenylsulfon*

1,6 g (0,005 Mol) 4,4'-Diamino-2-methylcarbonyl-diphenylsulfon (Beispiel 13) suspendiert in 75 ml 90%igem wässrigen Methanol werden mit 0,3 g (0,0075 Mol) Natriumborhydrid bei 20°C reduziert. Nach 30 Minuten erhält man eine klare Lösung. Man dampft das Methanol ab und verdünnt die wässrige Lösung mit weiterem Wasser. Dabei scheidet sich ein Öl ab, welches man mit Methylenchlorid extrahiert. Nach Abdampfen des Lösungsmittels verbleibt ein weisses, amorphes Produkt.

| | | | |
|---|---|---|---|
| NMR-Spektrum ($CDCl_3$): | 1,3 ppm | 2H | Dublett |
| | 5,5 ppm | 1H | Multiplett |
| 80 MHz | 6,6-7,9 ppm | 7H | Multiplett |

*Beispiel 15*

*4,4'-Diamino-2-cyano-diphenylsulfon*

6,3 g (0,021 Mol) 4,4'-Diamino-2-carbamoyl-diphenylsulfon (hergestellt analog Beispiel 18) werden in 45 ml Thionylchlorid 8 Stunden zum Rückfluss erhitzt. Man erhält eine gelborange Lösung und destilliert nun das Thionylchlorid ab. Es verbleibt ein gelber Schaum, welcher in Wasser aufgeschlämmt und mit Ammoniaklösung alkalisch gestellt wird. Die so erhaltene Base der Titelverbindung wird als amorphes, festes Produkt abgesaugt und mit Wasser gewaschen. Das Rohprodukt wird auf einer Kieselgelsäure chromatographisch gereinigt (Fliessmittel: 8 Teile Methylenchlorid, 1 Teil Methanol und 1 Teil Aceton). Die entsprechenden Fraktionen werden vom Fliessmittel befreit und ergeben ein amorphes, geblichweisses festes Produkt.

Massenspektrum ($CH_5$): $M^+$273 m/Z

*Beispiel 16*

*4,4'-Diamino-2-aminomethyl-diphenylsulfon*

0,35 g (0,0012 Mol) 4,4'-Diamino-2-carbamoyl--diphenylsulfon werden in 25 ml absolutem Tetrahydrofuran gelöst und mit 0,15 g (0,0036 Mol) Lithiumaluminiumhydrid versetzt. Man rührt eine Stunde bei 20°C und anschliessend erwärmt man während 8

Stunden auf 50°C. Das Lithiumaluminiumhydrid wird mit Eiswasser zersetzt und die Reaktionsmischung mit Äther extrahiert. Nach dem Abdampfen des Lösungsmittels hinterbleibt ein festes, gelbes Produkt, welches über eine Kieselgelsäule chromatographisch gereinigt wird (Fliessmittel: 6 Teile Methylenchlorid und 1 Teil Methanol). Nach Abdampfen des Fliessmittels der entsprechenden Fraktionen verbleibt ein gelbliches, amorphes Produkt.

Massenspektrum ($CH_5$): $M^+$277 m/Z

*Beispiel 17*

*4,4'-Diamino-2-hydroxymethyl-diphenylsulfon*

0,23 g (0,00075 Mol) 4,4'-Diamino-2-methoxycarbonyl-diphenylsulfon [J. med. chem. 14, 1168 (1971)] werden in 15 ml absolutem Tetrahydrofuran gelöst und mit 0,07 g (0,0019 Mol) Lithiumaluminiumhydrid versetzt. Man rührt eine Stunde bei 20°C und erwärmt anschliessend während 3 Stunden auf 50°C. Das überschüssige Lithiumaluminiumhydrid wird mit Eiswasser zersetzt und dann die Reaktionsmischung mit Äther extrahiert. Nach Abdampfen des Lösungsmittels verbleibt ein gelbbraunes Öl, welches über eine Kieselgelsäule chromatographisch gereinigt wird (Fliessmittel: 9 Teile Methylenchlorid und 1 Teil Methanol). Die entsprechenden Fraktionen ergeben nach Abdampfen des Fliessmittels ein gelbliches Öl.

Massenspektrum ($CH_5$): $M^+$278 m/Z

*Beispiel 18*

*4,4'-Diamino-2-(N-methylcarbamoyl)-diphenylsulfon*

0,95 g (0,0025 Mol) 4,4'-Diamino-2-chlorocarbonyl-diphenylsulfon-dihydrochlorid werden in 15 ml Methylenchlorid suspendiert und zu einer Lösung von 0,31 g (0,01 Mol) Methylamin in 20 ml Methylenchlorid gegeben. Es wird über Nacht bei 20°C gerührt. Anschliessend wird das Methylenchlorid abgedampft und der verbleibende feste Rückstand mit Wasser ausgewaschen. Es verbleibt ein weisser, amorpher Rückstand.

IR-Spektrum (KBr):

| | |
|---|---|
| 3480 + 3360 $cm^{-1}$ | $NH_2$ |
| 1630 $cm^{-1}$ | Amid |
| 1125 $cm^{-1}$ | $SO_2$ |

*Beispiel 19*

*2-(N-Cyclohexyl-N-methyl-carbamoyl)-4,4'-diamino-diphenylsulfon*

Hergestellt aus 4,4'-Diamino-2-chlorocarbonyl-diphenylsulfon-dihydrochlorid und N-Cyclohexyl-N-methylamin analog Beispiel 18.

IR-Spektrum ($CH_2Cl_2$):

| | |
|---|---|
| 3490, 3400 $cm^{-1}$ | $NH_2$ |
| 1620-1630 $cm^{-1}$ | Amid |
| 1300, 1140 $cm^{-1}$ | $SO_2$ |

*Beispiel 20*

*4,4'-Diamino-2-(N,N-dimethyl-amino)-diphenylsulfon*

*a) 2-Chlor-5-nitro-N,N-dimethy-anilin*

63 g Paraformaldehyd werden in 1 l 35%iger Ameisensäure vorgelegt und bei 100°C mit 60 g 2-Chlor-5-nitro-anilin in 560 ml 95%iger Ameisensäure portionsweise versetzt. Anschliessend wird 2 Stunden auf dem Dampfbad erhitzt, das Reaktionsgemisch eingeengt, mit 560 ml 2n NaOH und 140 g $Na_2SO_3$ versetzt, mit Methylenchlorid extrahiert, die organische Phase mit Magnesiumsulfat getrocknet, wieder zur Trockne eingeengt und über eine mit Kieselgel gefüllte Säule chromatographisch gereinigt (Fliessmittel: Dichlormethan/Cyclohexan = 2:1).

Gelbes Öl, das direkt weiter umgesetzt wird.

*b) 4'-Acetamino-2-(N,N-dimethyl-amino)-4-nitro-diphenylsulfon*

24 g 4-Acetylamino-phenylsulfinsäure-Natriumsalz, 22 g 2-Chlor-5-nitro-(N,N-dimethyl)-anilin und 120 ml absolutes Dimethylformamid werden zusammen gegeben, 28 Stunden unter Rückfluss gehalten, auf Wasser gegossen, mit Essigester extrahiert, die organische Phase mit Magnesiumsulfat getrocknet und im Vakuum eingeengt. Der Rückstand wird über eine mit Kieselgel gefüllte Säule gereinigt (Fliessmittel: Dichlormethan/Methanol = 50:1). Die Fraktionen, die das gewünschte Produkt enthalten, werden eingeengt, und der Rückstand wird mit Äther verrieben.

Gelbe Kristalle; Schmelzpunkt: 213-216°C.

*c) 4'-Amino-2-(N,N-dimethyl-amino)-4-nitro-diphenylsulfon*

10 g 4'-Acetamino-2-(N,N-dimethyl-amino)-4-nitro-diphenylsulfon werden in 50 ml halbkonz. Salzsäure vorgelegt und ca. eine Stunde auf dem Dampfbad bis zur klaren Lösung erhitzt. Dann wird abgekühlt, mit 2n Natronlauge basisch gestellt, mit Dichlormethan extrahiert, die organische Phase mit Magnesiumsulfat getrocknet und eingeengt. Der Rückstand wird über eine mit Kieselgel gefüllte Säule gereinigt (Fliessmittel: Dichlormethan/Methanol = 50:1).

Gelbe Kristalle; Schmelzpunkt: 164-166°C.

*d) 4,4'-Diamino-2-(N,N-dimethyl-amino)-diphenylsulfon*

6 g 4'-Amino-2-(N,N-dimethyl-amino)-4-nitro-diphenylsulfon werden in 200 ml Methanol mit 0,6 g Raney-Nickel während 6 Stunden bei Raumtemperatur und einem Druck von 3 bar hydriert. Anschliessend wird der Katalysator abgesaugt, das Filtrat im Vakuum zur Trockne eingeengt und über eine mit Kieselgel gefüllte Säule gereinigt (Fliessmittel: Dichlormethan/Methanol = 60:1).

Beige Kristalle; Schmelzpunkt: 196-197°C.

*Beispiel 21*

*4,4'-Diamino-2-(N-methyl-amino)-diphenylsulfon*

Hergestellt aus 2-Chlor-5-nitro-(N-methyl)-Anilin und 4-Acetylamino-phenylsulfinsäure-Natriumsalz analog Beispiel 20.

Erstarrter Schaum.

IR-Spektrum (Methylenchlorid):

3500, 3400 $cm^{-1}$ $NH_2$
1130, 1300 $cm^{-1}$ $SO_2$

*2-Chlor-5-nitro-N-methyl-anilin* wurde folgendermassen erhalten:

50 g 2-Chlor-5-nitro-anilin, 82 g Methyljodid und 123,3 g Natriumcarbonat werden in 1,2 l Äthanol 16 Stunden unter Rückfluss gekocht. Anschliessend wird der unlösliche Niederschlag abfiltriert, die Mutterlauge eingeengt, und der Rückstand wird in Dichlormethan aufgenommen. Die oroganische Phase wird mit Wasser gewaschen, mit Magnesiumsulfat getrocknet und im Vakuum eingeengt. Der Rückstand wird über eine mit Kieselgel gefüllte Säule gereinigt (Fliessmittel: Dichlormethan/Cyclohexan = 1:2).

Orange Kristalle; Schmelzpunkt: 106-108°C.

*Beispiel 22*

*4'-Amino-2,4-bis-(N,N-dimethyl-amino)-diphenylsulfon*

*a) 4'-Acetamino-4-amino-2-(N,N-dimethyl-amino)-diphenylsulfon*

4,5 g 4'-Acetamino-2-(N,N-dimethyl-amino)-4-nitro-diphenylsulfon werden in 200 ml Methanol mit 0,45 g Raney-Nickel während 2 Stunden bei Raumtemperatur und bei einem Druck von 3 bar hydriert. Anschliessend wird der Katalysator abfiltriert, das Filtrat eingeengt und der Rückstand über eine mit Kieselgel gefüllte Säule gereinigt (Fliessmittel: Dichlormethan/Methanol = 30:1).

Hellbraune Kristalle; Schmelzpunkt: 117-119°C.

*b) 4'-Acetamino-2,4-bis-(N,N-dimethyl-amino)-diphenylsulfon*

1,2 g Paraformaldehyd werden unter Erwärmen in 30 ml 95%iger Ameisensäure gelöst und 2,3 g 4'-Acetamino-4-amino-2-(N,N-dimethyl-amino)-diphenylsulfon zugegeben. Das Reaktionsgemisch wird 12 Stunden bei 90°C gehalten und dann im Vakuum eingeengt. Man versetzt mit 11 ml 2n Natronlauge und 2,7 g $Na_2SO_3$, extrahiert mit Essigester, behandelt den Extrakt mit Aktivkohle und Magnesiumsulfat, engt im Vakuum zur Trockne ein und reinigt den Rückstand über eine mit Kieselgel gefüllte Säule (Fliessmittel: Dichlormethan/Methanol = 30:1).

Farbloses Öl, das direkt weiter umgesetzt wird.

*c) 4'-Amino-2,4-bis-(N,N-dimethyl-amino)-diphenylsulfon*

1 g 4'-Acetamino-2,4-bis-(N,N-dimethyl-amino)-diphenylsulfon werden in 50 ml 2n Salzsäure gelöst und ca. 45 Minuten auf dem Dampfbad erhitzt. Anschliessend wird abgekühlt, mit 2n NaOH basisch gestellt und mit Methylenchlorid extrahiert. Die organische Phase wird mit Aktivkohle und Magnesiumsulfat behandelt, im Vakuum zur Trockne eingeengt, und der Rückstand wird über eine mit Kieselgel gefüllte Säule gereinigt (Fliessmittel: Dichlormethan/Methanol = 50:1). Der Rückstand der gewünschten Fraktionen wird mit Äther verrieben.

Gelbliche Kristalle; Schmelzpunkt: 166-168°C.

*Beispiel 23*

*4'-Amino-4-(N-äthyl-amino)-2-trifluormethyl-diphenylsulfon*

*a) 4'-Acetamino-4-nitro-2-trifluormethyl-diphenylsulfon*

50 g 4-Acetamino-phenylsulfinsäure-Natriumsalz und 48 g 2-Chlor-5-nitro-benzotrifluorid in 260 ml absolutem Dimethylformamid wurden ca. 25 Stunden unter Rückfluss gekocht. Anschliessend wird abgekühlt und auf ca. 2 Liter Wasser gegossen, mit Dichlormethan extrahiert, die organische Phase gut mit Wasser gewaschen, mit Magnesiumsulfat getrocknet und im Vakuum zur Trockne eingeengt. Der Rückstand wird mit Äther verrieben.

Hellgelbe Kristalle; Schmelzpunkt 228-230°C.

*b) 4'-Acetamino-4-amino-2-trifluormethyl-diphenylsulfon*

30 g 4'-Acetamino-4-nitro-2-trifluormethyl-diphenylsulfon werden in 800 ml Methanol und 3 g Raney-Nickel während 8 Stunden 40 Minuten bei Raumtemperatur und einem Druck von 3 bar hydriert. Anschliessend wird erwärmt, um das gewünschte Produkt wieder in Lösung zu bringen. Vom Katalysator wird warm abgesaugt, und das Filtrat wird im Vakuum eingeengt.

Beige Kristalle; Schmelzpunkt 220-224°C.

*c) 4'-Acetamino-4-(N-äthyl-amino)-2-trifluormethyl-diphenylsulfon*

3 g 4'-Acetamino-4-amino-2-trifluormethyl-diphenylsulfon, 6,7 g Acetaldehyd, 0,6 g geschmolzenes Natriumacetat und 3 g Raney-Nickel in 100 ml Äthanol werden bei 70°C und einem Druck von 5 bar während 11 Stunden hydriert. Anschliessend wird der Katalysator abfiltriert und das Filtrat im Vakuum zur Trockne eingeengt. Der Eindampfrückstand wird über eine mit Kieselgel gefüllte Säule chromatographisch gereinigt (Fliessmittel: Dichlormethan/Methanol = 50:1).

Farblose Kristalle; Schmelzpunkt: 157-159°C.

*d) 4'-Amino-4-(N-äthyl-amino)-2-trifluormethyl-diphenylsulfon*

1,5 g 4'-Acetamino-4-(-N-äthyl-amino)-2-trifluormethyl-diphenylsulfon werden in 20 ml halbkonzentrierter Salzsäure ca. 5 Minuten auf dem Dampfbad gerührt, bis sich eine klare Lösung einstellt. Anschliessend wird auf 50 ml Wasser gegossen, mit 2n Natronlauge basisch gestellt und der erhaltene Niederschlag abgesaugt. Dieser wird in Dichlormethan aufgenommen. Die Lösung wird mit Magnesiumsulfat getrocknet, im Vakuum zur Trockne eingeengt, und der Rückstand wird mit Äther verrieben.

Gelbliche Kristalle; Schmelzpunkt: 133-135°C.

*Beispiel 24*

*4'-Amino-4-äthylamino-2-cyan-diphenylsulfon*

*a) 4'-Acetamino-4-amino-2-methoxycarbonyl--diphenylsulfon*

Hergestellt aus 4'-Acetamino-2-methoxycarbonyl-4-nitro-diphenylsulfon (Lit.: J. med. Chem. 1971, 1168) durch katalytische Hydrierung analog Beispiel 23b.

IR-Spektrum (KBr):

| | |
|---|---|
| 3360, 3450 $cm^{-1}$ | $NH_2$ |
| 1735 $cm^{-1}$ | Ester-CO |
| 1650, 1680, 1550 $cm^{-1}$ | Amid-CO |

UV-(Methanol): 257 nm und 240 nm

*b) 4'-Acetamino-4-äthylamino-2-methoxycarbonyl-diphenylsulfon*

Hergestellt aus 4'-Acetamino-4-amino-2-methoxycarbonyl-diphenylsulfon, Acetaldehyd und katalytisch angeregtem Wasserstoff analog Beispiel 23c.

Amorphes Produkt.

IR-Spektrum ($CH_2Cl_2$):

| | |
|---|---|
| 3440 $cm^{-1}$ | -NH |
| 1740 $cm^{-1}$ | Ester-CO |
| 1710, 1520 $cm^{-1}$ | Amid-CO |

UV (Methanol): 260 nm, 300 nm

*c) 4-Äthylamino-4'-amino-2-methoxycarbonyl-diphenylsulfon*

Hergestellt aus 4'-Acetamino-4-äthylamino-2--methoxycarbonyl-diphenylsulfon und Salzsäure analog Beispiel 23d.

Schmelzpunkt: 155-156°C.

*d) 4-Äthylamino-4'-amino-2-carboxy-diphenylsulfon*

39 g (0,116 Mol) 4-Äthylamino-4'-amino-2--methoxycarbonyl-diphenylsulfon werden in 10 g Natriumhydroxid in einem Gemisch aus 70 ml Wasser und 250 ml Methanol 4 Stunden lang am Rückfluss gekocht. Nach Abkühlung wird von einer geringen Menge eines unlöslichen Materials abgesaugt; das Filtrat wird mit Salzsäure auf pH 4 gestellt, wobei das Reaktionsprodukt kristallin ausfällt. Es wird abgesaugt, mit Eiswasser gewaschen und getrocknet.

Schmelzpunkt: 116-122°C (Zers.).

*e) 4-Äthylamino-4'-amino-2-cyan-diphenylsulfon*

37 g (0,115 Mol) 4-Äthylamino-4'-amino-2-carboxy-diphenylsulfon werden in 370 ml Thionylchlorid 45 Minuten lang am Rückfluss gekocht. Man dampft im Vakuum zur Trockne ein und versetzt den Rückstand vorsichtig mit kaltem konzentriertem wässrigem Ammoniak. Man erhält 4-Äthylamino-4'-amino-carbamoyl-diphenylsulfon, das abgesaugt, mit Wasser gewaschen, getrocknet und ohne weitere Reinigung mit Thionylchlorid zum Nitril dehydratisiert wird. Dazu werden 32 g (etwa 0,1 Mol) des obigen Amids in 220 ml Thionylchlorid 2,75 Stunden am Rückfluss gekocht. Man engt das Reaktionsgemisch im Vakuum ein und reinigt das Rohprodukt durch wiederholte Chromatographie an Kieselgel (Elutionsmittel: Methylenchlorid/Methanol = 40:1).

Schmelzpunkt: 177-183°C.

*Beispiel 25*

*4'-Amino-2-methyl-4-n-propylamino-diphenylsulfon*

*a) 2-Methyl-4'-nitro-4-(N-n-propyl-N-tosyl-amino)-diphenylsulfon*

2,5 g 2-Methyl-4'-nitro-4-tosylamino-diphenylsulfon, 0,8 ml n-Propyljodid und 0,9 g wasserfreies Kaliumcarbonat werden in 30 ml Dimethylformamid unter Rühren während 17 Stunden auf 60°C erhitzt. Nach Abkühlen giesst man auf Wasser. Der ausgeschiedene Festkörper wird in Methylenchlorid aufgenommen. Die Methylenchlorid-Phase wird abgetrennt, mit Wasser gewaschen, über Natriumsulfat getrocknet und zur Trockne eingeengt. Beim Umkristallisieren des Rückstandes aus Petroläther/Essigester erhält man gelbe Kristalle vom Schmelzpunkt 132-135°C.

*b) 2-Methyl-4'-nitro-4-n-propylamino-diphenylsulfon*

Eine Lösung von 2,48 g 2-Methyl-4'-nitro-4-(N--n-propyl-N-tosyl-amino)-diphenylsulfon lässt man in 25 ml konzentrierter Schwefelsäure 4 Stunden bei Raumtemperatur stehen. Anschliessend wird in Eis/konzentrierte Ammoniumhydroxid-Lösung gegossen und der ausgeschiedene Festkörper mit Methylenchlorid extrahiert. Die Methylenchlorid-Phase wird mit Wasser gewaschen, über Natriumsulfat getrocknet und im Vakuum zur Trockne eingeengt und man erhält so die gewünschte Verbindung als gelben Festkörper.

*c) 4'-Amino-2-methyl-4-n-propylamino-diphenylsulfon*

1,6 g 2-Methyl-4'-nitro-4-n-propylamino-diphenylsulfon werden in 60 ml Methanol gelöst und bei Raumtemperatur in Gegenwart von 0,5 g Palladiumkohle (10%ig) bei einem Wasserstoffdruck von 50 psi hydriert. Nach beendeter Wasserstoff-Aufnahme (15 Minuten) wird vom Katalysator abfiltriert und die methanolische Lösung auf etwa 10 ml eingeengt. Die ausgeschiedenen farblosen Kristalle vom Schmelzpunkt 170-173°C werden abgesaugt.

*Beispiel 26*

*4'-Amino-4-cyclohexylamino-2-methyl-diphenylsulfon*

*a) 4-Cyclohexylamino-2-methyl-4'-nitro-diphenylsulfon*

2 g 4-Amino-2-methyl-4'-nitro-diphenylsulfon

werden in 120 ml Methylenchlorid gelöst. Zu dieser Lösung gibt man 15 g Molekularsieb A4 und 2 ml ätherischer Salzsäure und versetzt anschliessend unter Rühren mit 2 ml Cyclohexanon und 1 g Natriumcyanborhydrid. Man lässt über Nacht bei Raumtemperatur stehen und filtriert. Das Filtrat wird mit 50 ml 2n Salzsäure und anschliessend mit 200 ml 2n Ammoniak jeweils während 10 Minuten gerührt. Die Methylenchlorid-Phase wird abgetrennt, mit Wasser gewaschen, über Natriumsulfat getrocknet und eingeengt. Der feste gelbe Rückstand wird über Kieselgel mit Methylenchlorid als Elutionsmittel chromatographiert. Die Substanz enthaltenden Eluate werden eingeengt, und beim Umkristallisieren des Rückstandes aus Äther/Petroläther erhält man gelbe Kristalle vom Schmelzpunkt 116-119°C.

*b) 4'-Amino-4-cyclohexylamino-2-methyl-diphenylsulfon*

Hergestellt aus 4-Cyclohexylamino-2-methyl-4'-nitro-diphenylsulfon mit Wasserstoff und Palladiumkohle analog Beispiel 25c.

Schmelzpunkt: 180-183°C.

*Beispiel 27*

*4'-Amino-2-methyl-4-n-popylamino-diphenylsulfon*

1,5 g 2-Methyl-4'-nitro-4-propylidenamino-diphenylsulfon (hergestellt aus 4-Amino-2-methyl-4'-nitro-diphenylsulfon, Propionaldehyd, Titan(IV)-chlorid und Kaliumcarbonat in Dichlormethan) werden in 50 ml Methanol gelöst und in Gegenwart von 0,5 g Palladiumkohle bei Raumtemperatur und 50 psi Wasserstoffdruck bis zur beendeten Wasserstoff-Aufnahme hydriert. Nach Abtrennen des Katalysators wird die verbleibende Mutterlauge im Vakuum zur Trockne eingedampft. Der Rückstand wird über Kieselgel mit Methylenchlorid als Elutionsmittel chromatographiert. Beim Einengen der die Substanz enthaltenden Elutate erhält man farblose Kristalle vom Schmelzpunkt 170-173°C.

*Beispiel 28*

*4'-Amino-2-methyl-4-methylamino-diphenylsulfon*

Hergestellt aus 2-Methyl-4-methylamino-4'-nitro-diphenylsulfon mit Waserstoff und Palladiumkohle analog Beispiel 25c.

Schmelzpunkt: 150-153°C.

*Beispiel 29*

*4-Äthylamino-4'-amino-2-methyl-diphenylsulfon*

Hergestellt aus 4-Äthylamino-2-methyl-4'-nitro-diphenylsulfon mit Wasserstoff und Palladiumkohle analog Beispiel 25c.

Schmelzpunkt: 166-167°C.

*Beispiel 30*

*4'-Amino-4-isopropylamino-2-methyl-diphenylsulfon*

Hergestellt aus 4-Isopropylamino-2-methyl-4'-nitro-diphenylsulfon mit Wasserstoff und Palladiumkohle analog Beispiel 25c.

Schmelzpunkt: 149-150°C.

*Beispiel 31*

*4'-Amino-4-n-hexylamino-2-methyl-diphenylsulfon*

Hergestellt aus 4-n-Hexylamino-2-methyl-4'-nitro-diphenylsulfon mit Wasserstoff und Palladiumkohle analog Beispiel 25c.

Schmelzpunkt: 116-118°C.

*Beispiel 32*

*4'-Amino-4-cyclopentylamino-2-methyl-diphenylsulfon*

Hergestellt aus 4-Cyclopentylamino-2-methyl-4'-nitro-diphenylsulfon mit Wasserstoff und Palladiumkohle analog Beispiel 25c.

Schmelzpunkt: 176-178°C.

*Beispiel 33*

*4'-Amino-4-dimethylamino-2-methyl-diphenylsulfon*

Hergestellt aus 4-Dimethylamino-2-methyl-4'-nitro-diphenylsulfon mit Wasserstoff und Palladiumkohle analog Beispiel 25c.

Schmelzpunkt: 221-223°C.

*Beispiel 34*

*4'-Amino-4-n-hexylamino-2-methyl-diphenylsulfon*

Hergestellt aus 4'-Acetamino-4-(N-n-hexyl-N-tosyl-amino)-2-methyl-diphenylsulfon mit Phenol und Bromwasserstoffsäure analog Beispiel 6.

Schmelzpunkt: 116-118°C.

*Beispiel 35*

*4'-Amino-4-isopropylamino-2-methyl-diphenylsulfon*

Hergestellt aus 4'-Acetamino-4-(N-isopropyl-tosyl-amino)-2-methyl-diphenylsulfon mit Phenol und Bromwasserstoffsäure analog Beispiel 6.

Schmelzpunkt: 149-150°C.

*Beispiel 36*

*4'-Amino-2-chlor-4-cyclohexylamino-diphenylsulfon*

Hergestellt aus 4'-Acetamino-2-chlor-4-cyclohexylamino-diphenylsulfon und 3n Salzsäure analog Beispiel 22c.

Schmelzpunkt: 197-199°C.

*Beispiel 37*

Durch Hydrolyse einer entsprechenden Verbindung der allgemeinen Formel

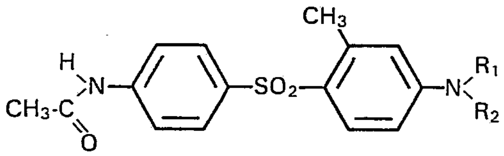

(IIIa)

erhält man folgende Verbindungen analog Beispiel 22c:

| 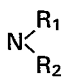 | Schmelzpunkt |
|---|---|
| $NHCH_3$ | 150-153°C |
| $NHC_2H_5$ | 166-167°C |
| $NHC_3H_7$ | 170-173°C |
| NH-iso$C_3H_7$ | 149-150°C |
| NH-$C_6H_{13}$ | 116-118°C |
| NH-Cyclopentyl | 176-178°C |
| NH-Cyclohexyl | 180-183°C |
| $N(CH_3)_2$ | 221-223°C |

*Beispiel 38*

*4-Äthylamino-4′-amino-2-hydroxymethyl-diphenylsulfon*

Hergestellt aus 4-Äthylamino-4′-amino-2-methoxycarbonyl-diphenylsulfon und Lithiumaluminiumhydrid analog Beispiel 17.
Schmelzpunkt: 148-151°C.

*Beispiel 39*

*4′-Amino-2-methylamino-4-n-propylamino-diphenylsulfon*

Hergestellt aus 4′-Acetamino-2-methylamino-4--n-propylamino-diphenylsulfon und 20%iger Salzsäure analog Beispiel 22c.
Schmelzpunkt: 142-143°C.

*Beispiel 40*

*4′-Amino-2-methyl-4-n-propylamino-diphenylsulfon*

0,8 g 4′-Amino-2-methyl-4-propionylamido-diphenylsulfon werden in 30 ml trockenem Tetrahydrofuran gelöst. Diese Lösung tropft man unter Rühren bei 0°C zu einer Suspension von 0,56 g Lithiumaluminiumhydrid in 20 ml trockenem Tetrahydrofuran. Anschliessend wird während 2 Stunden auf Siedetemperatur erhitzt. Nach Abkühlen wird überschüssiges Lithiumaluminiumhydrid mit wenig Wasser zerstört, 0,5 ml 10n Natriumhydroxid-Lösung zugesetzt und der ausgeschiedene Festkörper durch Absaugen über Kieselgur entfernt. Der Filterrückstand wird mit Tetrahydrofuran gewaschen, das Filtrat mit Natriumsulfat getrocknet und im Vakuum zur Trockne eingeengt.

Schmelzpunkt: 170-173°C (Äthanol).

*Beispiel I*

*Tabletten zu 100 mg 4,4′-Diamino-2-cyan-diphenylsulfon*

Zusammensetzung:

1 Tablette enthält:

| | |
|---|---|
| Wirksubstanz | 100,0 mg |
| Maisstärke, direkttablettierbar | 60,0 mg |
| Milchzucker, direkttablettierbar | 38,0 mg |
| Magnesiumstearat | 2,0 mg |
| | 200,0 g |

*Herstellung:*

Die Substanzen werden gleichmässig gemischt und zu Tabletten verpresst.

*Beispiel II*

*Tabletten zu 200 mg 4,4′-Diamino-2-cyan-diphenylsulfon*

1 Tablette enthält:

| | |
|---|---|
| Wirksubstanz | 200,0 mg |
| Maisstärke, direkttablettierbar | 120,0 mg |
| Milchzucker, direkttablettierbar | 76,0 mg |
| Magnesiumstearat | 4,0 mg |
| | 400,0 mg |

*Herstellung:* wie Beispiel I.

*Beispiel III*

*Dragées zu 50 mg 4,4′-Diamino-2-cyan-diphenylsulfon und 6,25 mg Pyrimethamin*

1 Dragéekern enthält:

| | |
|---|---|
| Wirksubstanz | 50,0 mg |
| Pyrimethamin | 6,25 mg |
| Maisstärke, direkttablettierbar | 40,0 mg |
| Milchzucker, direkttablettierbar | 22,75 mg |
| Magnesiumstearat | 1,0 mg |
| | 120,0 mg |

*Herstellung:* wie Beispiel I.

*Dragierung:*

Nach bekanntem Verfahren werden die Kerne mit einer Hülle überzogen, die im wesentlichen aus Zucker und Talkum besteht. Es werden 30 mg Hüllenmaterial verwendet.

*Beispiel IV*

*Tabletten zu 100 mg 4,4'-Diamino-2-cyan-diphenylsulfon und 12,5 mg Pyrimethamin*

1 Tablette enthält:

| | |
|---|---|
| Wirksubstanz | 100,0 mg |
| Pyrimethamin | 12,5 mg |
| Maisstärke, direkttablettierbar | 60,0 mg |
| Milchzucker, direkttablettierbar | 25,5 mg |
| Magnesiumstearat | 2,0 mg |
| | 200,0 mg |

*Herstellung:* wie Beispiel I.

*Beispiel V*

*Ampulen zu 100 mg 4,4'-Diamino-2-cyan-diphenylsulfon (i.m. u. s.c.-Kristallsuspension)*

1 Ampulle enthält:

| | | |
|---|---|---|
| Wirksubstanz | | 100,9 mg |
| Natriumchlorid | | 15,0 mg |
| Aqua bidest. | ad | 3,0 ml |

*Herstellung:*

Die Wirksubstanz wird fein gemahlen (Teilchengrösse < 5 µm). In der Natriumchlorid-Lösung wird die Substanz homogen verteilt. Die Suspension wird in 3-ml-Ampullen abgefüllt und 20 Minuten bei 121 °C sterilisiert.

*Beispiel VI*

*Ampullen zu 100 mg 4,4'-Diamino-2-cyan-diphenylsulfon (i.m. u. s.c.-Kristallsuspension) und 12,5 mg Pyrimethamin (Kombination mit Pyrimethamin)*

1 Ampulle enthält:

| | | |
|---|---|---|
| Wirksubstanz | | 100,0 mg |
| Pyrimethamin | | 12,5 mg |
| Natriumchlorid | | 15,0 mg |
| Aqua bidest. | ad | 3,0 ml |

*Herstellung:*

Die Wirksubstanzen werden fein gemahlen (Teilchengrösse < 5 µm). In der Natriumchlorid-Lösung werden die Substanzen homogen verteilt. Die Suspension wird in 3-ml-Ampullen abgefüllt und 20 Minuten bei 121 °C sterilisiert.

**Patentansprüche**

1. Neue substituierte Bis(4-aminophenyl)-sulfone der allgemeinen Formel

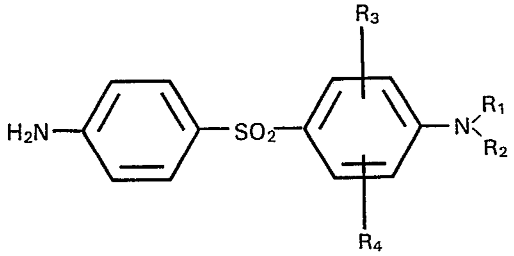

(I)

in der

$R_1$ ein Wasserstoffatom, eine Alkylgruppe mit 1 bis 7 Kohlenstoffatomen oder eine Cycloalkylgruppe mit 3 bis 7 Kohlenstoffatomen,

$R_2$ ein Wasserstoffatom oder eine Alkylgruppe mit 1 bis 3 Kohlenstoffatomen,

$R_3$ eine Nitril-, Alkylaminocarbonyl-, Dialkylaminocarbonyl-, N-Cycloalkyl-alkylaminocarbonyl-, Alkylamino-, Dialkylamino-, Dialkylaminocarbonylalkoxy-, Alkylaminosulfonyl-, Dialkylaminosulfonyl-, Hydroxyalkyl-, Alkylcarbonyl-, Aminoalkyl- oder Alkoxyalkylgruppe

oder, wenn $R_1$ und $R_2$ jeweils ein Wasserstoffatom darstellen, auch eine Hydroxy- oder Hydroxycarbonyl-alkoxygruppe

oder, wenn $R_1$ eine Alkyl- oder Cycloalkylgruppe und $R_2$ ein Wasserstoffatom oder eine Alkylgruppe darstellen, auch ein Halogenatom, eine Trifluormethyl-, Nitro-, Amino-, Aminosulfonyl-, Aminocarbonyl-, Alkyl-, Carboxy- oder Alkoxycarbonylgruppe und

$R_4$ ein Wasserstoffatom oder, wenn $R_1$ und $R_2$ jeweils ein Wasserstoffatom und $R_3$ in 2-Stellung ein Halogenatom oder eine Hydroxygruppe darstellen, auch ein Halogenatom, eine Hydroxy- oder Alkoxygruppe bedeuten,

wobei die bei der Definition der Reste $R_3$ und $R_4$ vorstehend erwähnten Alkylteile jeweils 1 bis 3 Kohlenstoffatome und der Cycloalkylteil 3 bis 7 Kohlenstoffatome enthalten kann, und deren Säureadditionssalze.

2. Neue Verbindungen der allgemeinen Formel I gemäss Anspruch 1, in der

$R_1$ ein Wasserstoffatom, eine Alkylgruppe mit 1 bis 7 Kohlenstoffatomen oder eine Cycloaklylgruppe mit 4 bis 7 Kohlenstoffatomen,

$R_2$ ein Wasserstoffatom oder eine Methylgruppe,

$R_3$ eine Nitril-, Methylaminocarbonyl-, N-Cyclohexyl-methyl-aminocarbonyl-, Methylamino-, Dimethylamino-, Dimethylaminocarbonylmethoxy-, Hydroxymethyl-, Hydroxyäthyl-, Methylcarbonyl-, Aminocarbonyl- oder Methoxymethylgruppe

oder, wenn $R_1$ und $R_2$ jeweils ein Wasserstoffatom darstellen, auch eine Hydroxy- oder Hydroxycarbonylmethoxygruppe

oder, wenn $R_1$ eine Alkyl- oder Cycloalkylgruppe und $R_2$ ein Wasserstoffatom oder eine Methylgruppe darstellen, auch ein Chlor- oder Bromatom, eine Methyl-, Trifluormethyl-, Nitro-, Amino- oder Aminocarbonylgruppe und

$R_4$ ein Wasserstoffatom oder, wenn $R_1$ und $R_2$ jeweils ein Wasserstoffatom und $R_3$ in 2-Stellung eine Hydroxgruppe, ein Chlor- oder Bromatom darstellen, auch ein Chlor- oder Bromatom, eine Hydroxy- oder Methoxygruppe bedeuten, und deren Säureadditionssalze.

3. Neue Verbindungen der allgemeinen Formel I gemäss Anspruch 1, in der

$R_1$ ein Wasserstoffatom, eine Alkylgruppe mit 1 bis 3 Kohlenstoffatomen oder eine Cycloalkylgruppe mit 4 bis 7 Kohlenstoffatomen,

$R_2$ ein Wasserstoffatom oder, wenn $R_1$ eine Methylgruppe darstellt, auch eine Methylgruppe,

$R_3$ in 2-Stellung ein Chlor- oder Bromatom, eine Methyl-, Trifluormethyl-, Hydroxymethyl-, Hydroxyäthyl-, Methylamino-, Dimethylamino-, Cyano- oder Methylcarbonylgruppe und

$R_4$ ein Wasserstoffatom bedeuten, und deren Säureadditionssalze.

4. 4-Amino-2-methyl-4-n-propylamino-diphenylsulfon und dessen Säureadditionssalze.

5. 4-Äthylamino-4'-amino-2-chlor-diphenylsulfon und dessen Säureadditionssalze.

6. Physiologisch verträgliche Säureadditionssalze der Verbindungen gemäss den Ansprüchen 1 bis 5 mit anorganischen oder organischen Säuren.

7. Arzneimittel, enthaltend eine Verbindung gemäss den Ansprüchen 1 bis 5 oder ein physiologisch verträgliches Säureadditionssalz gemäss Anspruch 6 neben einem oder mehreren inerten Trägerstoffen und/oder Verdünnungsmitteln.

8. Eine Verbindung gemäss den Ansprüchen 1 bis 5 oder ein physiologisch verträgliches Säureadditionssalz hiervon gemäss Anspruch 6 zur Behandlung von bakteriellen Erkrankungen, der Malaria oder Lepra.

9. Verfahren zur Herstellung eines Arzneimittels zur Behandlung von bakteriellen Erkrankungen, der Malaria und der Lepra, dadurch gekennzeichnet, dass eine Verbindung gemäss den Ansprüchen 1 bis 5 oder ein physiologisch verträgliches Säureadditionssalz hiervon gemäss Anspruch 6 auf nichtchemischem Wege in einen oder mehrere inerte übliche Trägerstoffe und/oder Verdünnungsmittel eingearbeitet wird.

10. Verfahren zur Herstellung der Verbindungen gemäss den Ansprüchen 1 bis 6, dadurch gekennzeichnet, dass

a.) eine Verbindung der allgemeinen Formel

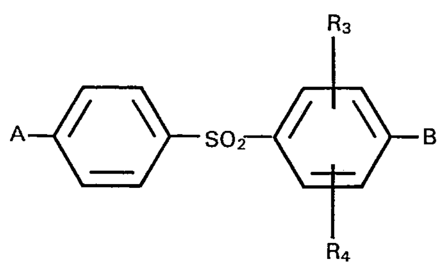

(II)

in der

$R_3$ und $R_4$ wie in den Ansprüchen 1 bis 5 definiert sind, einer der Reste A oder B eine Nitrogruppe und der andere der Reste A oder B eine $\genfrac{}{}{0pt}{}{R_1}{R_2}{>}N$-Gruppe, wobei $R_1$ und $R_2$ wie in den Ansprüchen 1 bis 5 definiert sind, oder ebenfalls eine Nitrogruppe darstellt, reduziert wird oder

b.) ein oder zwei Schutzreste von einer Verbindung der allgemeinen Formel

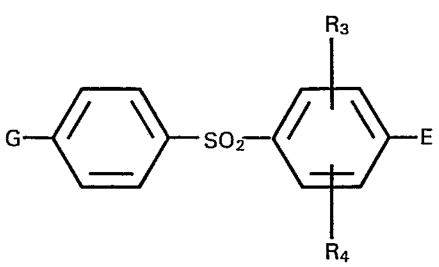

(III)

in der

$R_3$ und $R_4$ wie in den Ansprüchen 1 bis 5 definiert sind,

E eine durch einen Schutzrest geschützte Amino-, Alkylamino- oder Cycloalkylaminogruppe, wobei der Alkylteil 1 bis 7 Kohlenstoffatome und der Cycloalkylteil 3 bis 7 Kohlenstoffatome enthalten kann, oder eine $\genfrac{}{}{0pt}{}{R_1}{R_2}{>}N$-Gruppe, wobei $R_1$ und $R_2$ wie in den Ansprüchen 1 bis 5 definiert sind, und

G eine gegebenenfalls durch einen Schutzrest geschützte Aminogruppe bedeuten, wobei jedoch mindestens einer der Rest E oder G eine der oben erwähnten durch einen Schutzrest geschützte Gruppe darstellen muss, abgespalten wird oder

c.) zur Herstellung von Verbindungen der allgemeinen Formel I, in der $R_3$ eine Cyanogruppe darstellt, eine Verbindung der allgemeinen Formel

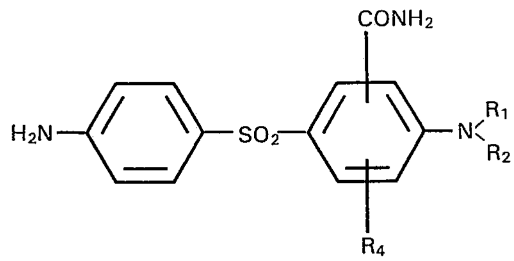

(IV)

in der

$R_1$, $R_2$ und $R_4$ wie in den Ansprüchen 1 bis 5 definiert sind, dehydratisiert wird oder

d.) zur Herstellung von Verbindungen der allgemeinen Formel I, in der $R_3$ eine Hydroxycarbonyl-alkoxy- oder Dialkylaminocarbonyl-alkoxygruppe darstellt, eine Verbindung der allgemeinen Formel

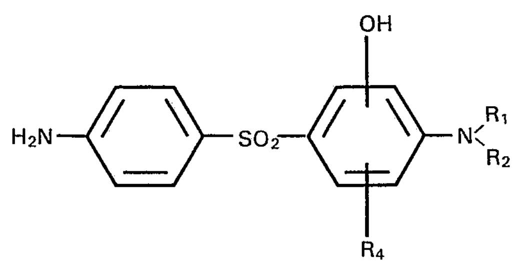

(V)

in der

$R_1$, $R_2$ und $R_4$ wie in den Ansprüchen 1 bis 5 definiert sind, mit einer Verbindung der allgemeinen Formel

$$X - Alk - CO - R_5 \qquad (VI)$$

in der

Alk eine Alkylengruppe mit 1 bis 3 Kohlenstoffatomen,

$R_5$ eine Hydroxy-, Alkoxy- oder Dialkylaminogruppe, wobei der Alkylteil jeweils 1 bis 3 Kohlenstoffatome enthalten kann, und

X eine nukleophile Austrittsgruppe wie ein Chlor- oder Bromatom bedeuten, alkyliert und gegebenenfalls anschliessend hydrolysiert wird oder

e.) zur Herstellung von Verbindungen der allgemeinen Formel I, in der $R_3$ eine Aminocarbonyl-, Al-

kylaminocarbonyl-, Dialkylaminocarbonyl- oder Dialkylaminocarbonyl-alkoxygruppe darstellt, eine Verbindung der allgemeinen Formel

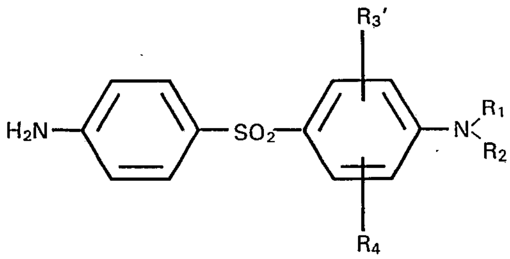
(VII)

in der

$R_1$, $R_2$ und $R_4$ wie in den Ansprüchen 1 bis 5 definiert sind und

$R_3'$ eine Hydroxycarbonyl- oder Hydroxycarbonylalkoxygruppe darstellt, oder deren reaktionsfähige Derivate mit einem Amin der allgemeinen Formel

$$H-N{<}^{R_6}_{R_7} \qquad (VIII)$$

in der

$R_6$ ein Wasserstoffatom oder eine Alkylgruppe mit 1 bis 3 Kohlenstoffatomen und

$R_7$ ein Wasserstoffatom oder eine Alkylgruppe mit 1 bis 3 Kohlenstoffatomen bedeuten, oder dessen reaktionsfähigen Derivaten umgesetzt wird oder

f.) zur Herstellung von Verbindungen der allgemeinen Formel I, in der $R_3$ eine Hydroxymethyl-, Aminomethyl- oder 1-Hydroxyalkylgruppe darstellt, eine Verbindung der allgemeinen Formel

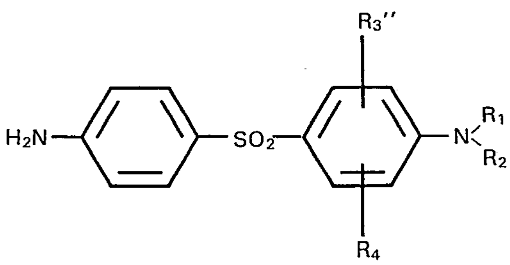
(IX)

in der

$R_1$, $R_2$ und $R_4$ wie in den Ansprüchen 1 bis 5 definiert sind und

$R_3''$ eine Hydroxycarbonyl-, Alkoxycarbonyl-, Aminocarbonyl- oder Alkylcarbonylgruppe darstellt, wobei die Alkylcarbonylgruppe insgesamt 2 bis 4 Kohlenstoffatome enthalten kann, reduziert wird oder

g.) zur Herstellung von Verbindungen der allgemeinen Formel I, in der $R_1$ eine Alkyl- oder Cycloalkylgruppe und $R_2$ ein Wasserstoffatom darstellen, eine Verbindung der allgemeinen Formel

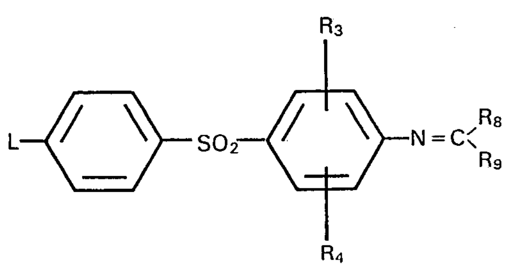
(X)

in der

$R_3$ und $R_4$ wie in den Ansprüchen 1 bis 5 definiert sind,

L eine Amino- oder Nitrogruppe und

$R_8$ und $R_9$ zusammen mit dem dazwischenliegenden Kohlenstoffatom eine Alkylidengruppe mit 1 bis 7 Kohlenstoffatomen oder eine Cycloalkylidengruppe mit 3 bis 7 Kohlenstoffatomen bedeuten, reduziert wird oder

h.) zur Herstellung von Verbindungen der allgemeinen Formel I, in der mindestens einer der Reste $R_1$ oder $R_2$ kein Wasserstoffatom darstellt und $R_3$ ein Halogenatom oder eine Alkylamino-, Dialkylamino-, Hydroxyalkyl-, Aminoalkyl-, Alkoxyalkyl-, Hydroxy-, Amino-, Trifluormethyl- oder Alkylgruppe bedeutet, eine Verbindung der allgemeinen Formel

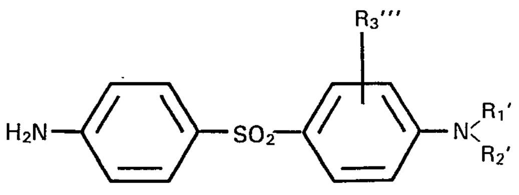
(XI)

in der

$R_1'$ und $R_2'$ die für $R_1$ und $R_2$ in den Ansprüchen 1 bis 5 erwähnten Bedeutungen besitzt, wobei jedoch einer der Reste $R_1'$ oder $R_2'$ eine Alkanoylgruppe mit 1 bis 7 Kohlenstoffatomen darstellen muss, und

$R_3'''$ ein Halogenatom oder eine Alkylamino-, Dialkylamino-, Hydroxyalkyl-, Aminoalkyl-, Alkoxyalkyl-, Hydroxy-, Amino-, Trifluormethyl- oder Alkylgruppe, wobei der Alkylteil jeweils 1 bis 3 Kohlenstoffatome enthalten kann, bedeutet, reduziert wird und

gewünschtenfalls eine so erhaltene Verbindung der allgemeinen Formel I, in der $R_3$ eine Alkoxycarbonyl- oder Alkoxycarbonyl-alkoxygruppe darstellt, mittels Hydrolyse in eine entsprechende Verbindung der allgemeinen Formel I, in der $R_3$ eine Hydroxycarbonyl- oder Hydroxycarbonyl-alkoxygruppe darstellt, übergeführt wird oder

eine so erhaltene Verbindung der allgemeinen Formel I, in der $R_3$ und/oder $R_4$ ein Chlor- oder Bromatom darstellten, mittels Hydrolyse oder Alkoholyse in eine entsprechende Verbindung der allgemeinen Formel I, in der $R_3$ in 2-Stellung eine Hydroxy- oder Alkoxygruppe und $R_4$ ein Chlor- oder Bromatom darstellen, übergeführt wird oder

eine so erhaltene Verbindung der allgemeinen Formel I in ihr Säureadditionssalz, insbesondere in ihr physiologisch verträgliches Säureadditionssalz, mit anorganischen oder organischen Säuren übergeführt wird.

11. Arzneimittel, enthaltend eine Verbindung gemäss den Ansprüchen 1 bis 5 oder ein physiologisch verträgliches Säureadditionssalz gemäss Anspruch 6 und einen Dihydrofolsäure-Reduktase-Hemmer neben einem oder mehreren inerten Trägerstoffen und/oder Verdünnungsmitteln.

12. Verfahren zur Herstellung eines Arzneimittels zur Behandlung von bakteriellen Erkrankungen, der Malaria und der Lepra, dadurch gekennzeichnet, dass eine Verbindung gemäss den Ansprüchen 1 bis

5 oder ein physiologisch verträgliches Säureadditionssalz hiervon gemäss Anspruch 6 und ein Dihydrofolsäure-Reduktase-Hemmer auf nichtchemischem Wege in einen oder mehrere inerte übliche Trägerstoffe und/oder Verdünnungsmittel eingearbeitet wird.

## Claims

1. New substituted bis(4-aminophenyl)-sulfones of general formula

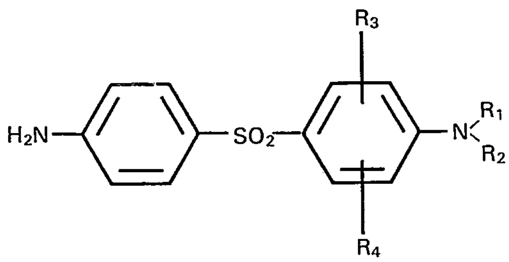

(I)

wherein

$R_1$ represents a hydrogen atom, an alkyl group with 1 to 7 carbon atoms or a cycloalkyl group with 3 to 7 carbon atoms,

$R_2$ represents a hydrogen atom or an alkyl group with 1 to 3 carbon atoms,

$R_3$ represents a nitrile, alkylaminocarbonyl, dialkylaminocarbonyl, N-cycloalkyl-alkylaminocarbonyl, alkylamino, dialkylamino, dialkylaminocarbonylalkoxy, alkylaminosulfonyl, dialkylaminosulfonyl, hydroxyalkyl, alkylcarbonyl, aminoalkyl or alkoxyalkyl group

or, if $R_1$ and $R_2$ represent hydrogen atoms, a hydroxy or hydroxycarbonylalkoxy group

or, if $R_1$ represents an alkyl or cycloalkyl group and $R_2$ represents a hydrogen atom or an alkyl group, it may also represent a halogen atom or a trifluoromethyl, nitro, amino, aminosulfonyl, aminocarbonyl, alkyl, carboxy or alkoxycarbonyl group and

$R_4$ represents a hydrogen atom or, if $R_1$ and $R_2$ both represent hydrogen atoms and $R_3$ in the 2-position represents a halogen atom or a hydroxy group, $R_4$ may also represent a halogen atom or a hydroxy or alkoxy group,

whilst the alkyl parts mentioned in the definitions of the groups $R_3$ and $R_4$ hereinbefore may each contain 1 to 3 carbon atoms and the cycloalkyl part may contain from 3 to 7 carbon atoms,

and the acid addition salts thereof.

2. New compounds of general formula I as claimed in claim 1, wherein

$R_1$ represents a hydrogen atom, an alkyl group containing 1 to 7 carbon atoms or a cycloalkyl group containing 4 to 7 carbon atoms,

$R_2$ represents a hydrogen atom or a methyl group,

$R_3$ represents a nitrile, methylaminocarbonyl, N-cyclohexyl-methylaminocarbonyl, methylamino, dimethylamino, dimethylaminocarbonylmethoxy, hydroxymethyl, hydroxyethyl, methylcarbonyl, aminocarbonyl or methoxymethyl group

or, if $R_1$ and $R_2$ both represent hydrogen atoms, $R_3$ may also represent a hydroxy or hydroxycarbonylmethoxy group

or, if $R_1$ represents an alkyl or cycloalkyl group and $R_2$ represents a hydrogen atom or a methyl group, $R_3$ may also represent a chlorine or bromine atom or a methyl, trifluoromethyl, nitro, amino or aminocarbonyl group and

$R_4$ represents a hydrogen atom or, if $R_1$ and $R_2$ both represent hydrogen atoms and $R_3$ in the 2-position represents a hydroxy group or a chlorine or bromine atom, $R_4$ may also represent a chlorine or bromine atom or a hydroxy or methoxy group,

and the acid addition salts thereof.

3. New compounds of general formula I as claimed in claim 1, wherein

$R_1$ represents a hydrogen atom, an alkyl group with 1 to 3 carbon atoms or a cycloalkyl group with 4 to 7 carbon atoms,

$R_2$ represents a hydrogen atom or, if $R_1$ represents a methyl group, $R_2$ may also represent a methyl group,

$R_3$ in the 2 position represents a chlorine or bromine atom or a methyl, trifluoromethyl, hydroxymethyl, hydroxyethyl, methylamino, dimethylamino, cyano or methylcarbonyl group and

$R_4$ represents a hydrogen atom,

and the acid addition salts thereof.

4. 4'-Amino-2-methyl-4-n-propylamino-diphenylsulfone and the acid addition salts thereof.

5. 4-Ethylamino-4'-amino-2-chloro-diphenylsulfone and the acid addition salts thereof.

6. Physiologically acceptable acid addition salts of the compounds as claimed in claims 1 to 5 with inorganic or organic acids.

7. Pharmaceutical compositions containing a compound as claimed in claims 1 to 5 or a physiologically acceptable acid addition salt as claimed in claim 6 together with one or more inert carriers and/or diluents.

8. A compound as claimed in claims 1 to 5 or a physiologically acceptable acid addition salt thereof as claimed in claim 6 for the treatment of bacterial diseases, malaria and leprosy.

9. Process for preparing a pharmaceutical composition for treating bacterial diseases, malaria and leprosy, characterised in that a compound as claimed in claims 1 to 5 or a physiologically acceptable acid addition salt thereof as claimed in claim 6 is incorporated in a non-chemical manner in one or more inert conventional carriers and/or diluents.

10. Process for preparing the compounds as claimed in claims 1 to 6, characterised in that

a) a compound of general formula

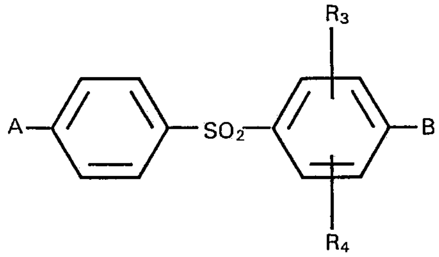

(II)

wherein

$R_3$ and $R_4$ are defined as in claims 1 to 5,

one of the groups A or B represents a nitro group and the other group A or B represents a $\begin{matrix}R_1\\R_2\end{matrix}\!>N$ group, wherein $R_1$ and $R_2$ are defined as in claims 1 to 5, or also represents a nitro group, is reduced, or

b) one or two protecting groups are split off from a compound of general formula

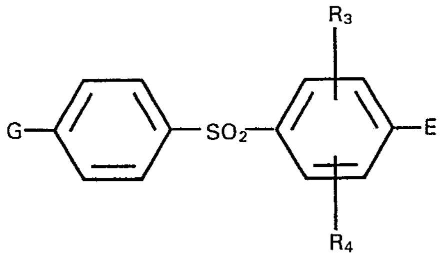

(III)

wherein

$R_3$ and $R_4$ are defined as in claims 1 to 5,

E represents an amino, alkylamino or cycloalkylamino group protected by a protecting group, whilst the alkyl part may contain from 1 to 7 carbon atoms and the cycloalkyl part may contain from 3 to 7 carbon atoms, or a $\begin{matrix}R_1\\R_2\end{matrix}\!>N$ group, wherein $R_1$ and $R_2$ are defined as in claims 1 to 5, and

G represents an amino group optionally protected by a protecting group, but at least one of the groups E or G must represent one of the above-mentioned groups protected by a protecting group, or

c) in order to prepare compounds of general formula I wherein $R_3$ represents a cyano group, a compound of general formula

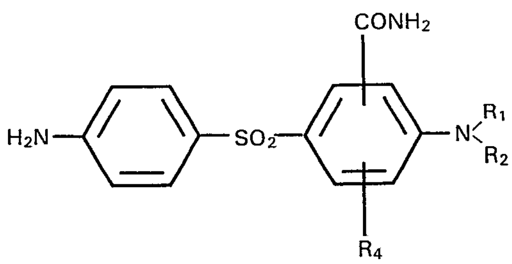

(IV)

wherein

$R_1$, $R_2$ and $R_4$ are defined as in claims 1 to 5, is dehydrated, or

d) in order to prepare compounds of general formula I wherein $R_3$ represents a hydroxycarbonylalkoxy or dialkylaminocarbonylalkoxy group, a compound of general formula

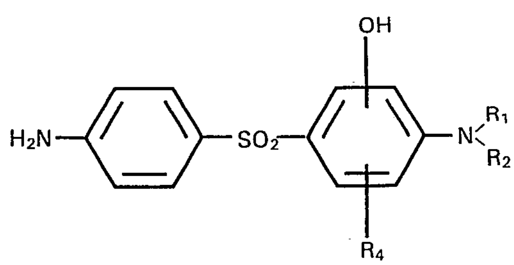

(V)

(wherein $R_1$, $R_2$ and $R_4$ are defined as in claims 1 to 5) is alkylated with a compound of general formula

$X - Alk - CO - R_5$ (VI)

(wherein Alk represents an alkylene group with 1 to 3 carbon atoms,

$R_5$ represents a hydroxy, alkoxy or dialkylamino group, wherein the alkyl part may contain 1 to 3 carbon atoms, and

X represents a nucleophilically exchangeable group such as a chlorine or bromine atom) and subsequently, if desired, hydrolysed, or

e) in order to prepare compounds of general formula I wherein $R_3$ represents an aminocarbonyl, alkylaminocarbonyl, dialkylaminocarbonyl or dialkylaminocarbonyl-alkoxy group, a compound of general formula

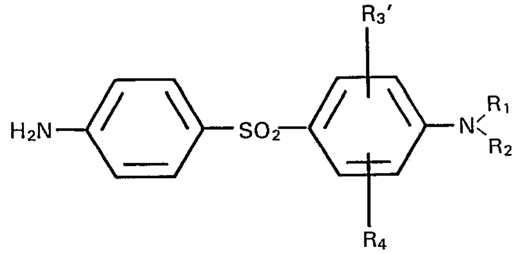

(VII)

(wherein $R_1$, $R_2$ and $R_4$ are defined as in claims 1 to 5 and $R_3'$ represents a hydroxycarbonyl or hydroxycarbonylalkoxy group), or a reactive derivative thereof, is reacted with an amine of general formula

$H - N\!<\!\begin{matrix}R_6\\R_7\end{matrix}$ (VIII)

wherein $R_6$ represents a hydrogen atom or an alkyl group with 1 to 3 carbon atoms and

$R_7$ represents a hydrogen atom or an alkyl group with 1 to 3 carbon atoms, or the reactive derivatives thereof, or

f) in order to prepare compounds of general formula I wherein $R_3$ represents a hydroxymethyl, aminomethyl or 1-hydroxyalkyl group, a compound of general formula

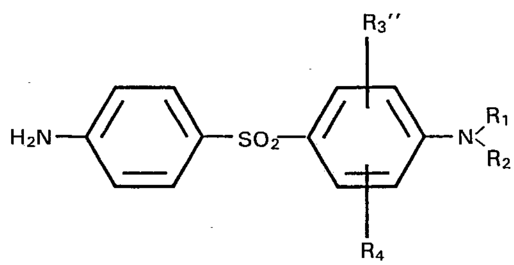

(IX)

wherein

$R_1$, $R_2$ and $R_4$ are defined as in claims 1 to 5 and

$R_3''$ represents a hydroxycarbonyl, alkoxycarbonyl, aminocarbonyl or alkylcarbonyl group, wherein the alkylcarbonyl group may contain a total of 2 to 4 carbon atoms, is reduced, or

g) in order to prepare compounds of general formula I wherein $R_1$ represents an alkyl or cycloalkyl

group and $R_2$ represents a hydrogen atom, a compound of general formula

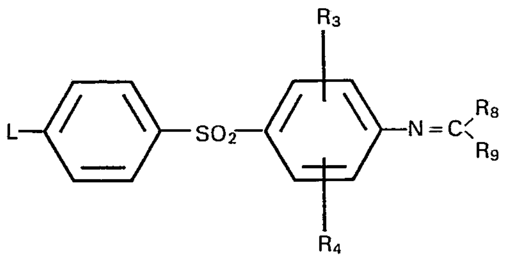

(X)

wherein

$R_3$ and $R_4$ are defined as in claims 1 to 5,

L represents an amino or nitro group and

$R_8$ and $R_9$ together with the carbon atom between them represent an alkylidene group with 1 to 7 carbon atoms or a cycloalkylidene group with 3 to 7 carbon atoms, is reduced, or

h) in order to prepare compounds of general formula I wherein at least one of the groups $R_1$ or $R_2$ does not represent a hydrogen atom and $R_3$ represents a halogen atom or an alkylamino, dialkylamino, hydroxyalkyl, aminoalkyl, alkoxyalkyl, hydroxy, amino, trifluoromethyl or alkyl group, a compound of general formula

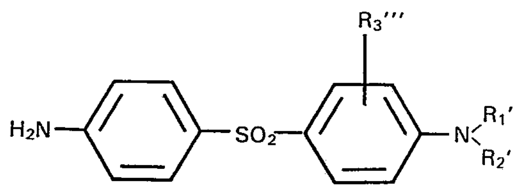

(XI)

wherein

$R_1'$ and $R_2'$ have the meanings given for $R_1$ and $R_2$ in claims 1 to 5, but one of the groups $R_1'$ or $R_2'$ must represent an alkanoyl group with 1 to 7 carbon atoms, and

$R_3'''$ represents a halogen atom or an alkylamino, dialkylamino, hydroxyalkyl, aminoalkyl, alkoxyalkyl, hydroxy, amino, trifluoromethyl or alkyl group, whilst the alkyl part may contain 1 to 3 carbon atoms, is reduced, and

if desired a compound of general formula I thus obtained wherein $R_3$ represents an alkoxycarbonyl or alkoxycarbonyl-alkoxy group is converted by hydrolysis into a corresponding compound of general formula I wherein $R_3$ represents a hydroxycarbonyl or hydroxycarbonyl-alkoxy group,

or a compound of general formula I thus obtained wherein $R_3$ and/or $R_4$ represent a chlorine or bromine atom is converted by hydrolysis or alcoholysis into a corresponding compound of general formula I wherein $R_3$ in the 2-position represents a hydroxy or alkoxy group and $R_4$ represents a chlorine or bromine atom, or

a compound of general formula I thus obtained is converted into an acid addition salt thereof, more particularly a physiologically acceptable acid addition salt, with inorganic or organic acids.

11. Pharmaceutical compositions containing a compound as claimed in claims 1 to 5 or a physiologically acceptable acid addition salt as claimed in claim 6 and a dihydrofolic acid reductase inhibitor together with one or more inert carriers and/or diluents.

12. Process for preparing a pharmaceutical composition for the treatment of bacterial diseases, malaria and leprosy, characterised in that a compound as claimed in claims 1 to 5 or a physiologically acceptable acid addition salt thereof as claimed in claim 6 and a dihydrofolic acid-reductase inhibitor is incorporated, by a non-chemical method, in one or more inert conventional carriers and/or diluents.

**Revendications**

1. Nouvelles bis(4-aminophényl)-sulfones substituées de formule générale

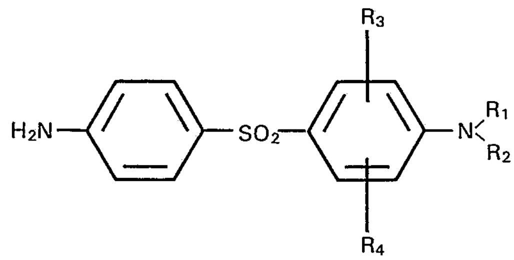

(I)

dans laquelle

$R_1$ représente un atome d'hydrogène, un groupe alcoyle avec 1 à 7 atomes de carbone ou un groupe cycloalcoyle avec 3 à 7 atomes de carbone,

$R_2$ représente un atome d'hydrogène ou un groupe alcoyle avec 1 à 3 atomes de carbone,

$R_3$ représente un groupe nitrile, alcoylaminocarbonyle, dialcoylaminocarbonyle, N-cycloalcoyl-alcoylaminocarbonyle, alcoylamino, dialcoylamino, dialcoylaminocarbonyl-alcoxy, alcoylaminosulfonyle, dialcoylaminosulfonyle, hydroxyalcoyle, alcoylcarbonyle, aminoalcoyle ou alcoxyalcoyle

ou, lorsque $R_1$ et $R_2$ représentent chacun un atome d'hydrogène, également un groupe hydroxy ou hydroxycarbonyl-alcoxy

ou, lorsque $R_1$ représente un groupe alcoyle ou cycloalcoyle et $R_2$ un atome d'hydrogène ou un groupe alcoyle, également un atome d'halogène, un groupe trifluorométhyle, nitro, amino, aminosulfonyle, aminocarbonyle, alcoyle, carboxy ou alcoxycarbonyle, et

$R_4$ représente un atome d'hydrogène ou, lorsque $R_1$ et $R_2$ représentent chacun un atome d'hydrogène et $R_3$ en position 2 un atome d'halogène ou un groupe hydroxy, également un atome d'halogène ou un groupe hydroxy ou alcoxy,

dans la définition des radicaux $R_3$ et $R_4$, les parties alcoyle mentionnées plus haut pouvant contenir chacune 1 à 3 atomes de carbone et la partie cycloalcoyle 3 à 7 atomes de carbone,

et leurs sels d'addition d'acides.

2. Nouveaux composés de formule générale I selon la revendication 1, dans laquelle

$R_1$ représente un atome d'hydrogène, un groupe

alcoyle avec 1 à 7 atomes de carbone ou un groupe cycloalcoyle avec 4 à 7 atomes de carbone,

$R_2$ représente un atome d'hydrogène ou un groupe méthyle,

$R_3$ représente un groupe nitrile, méthylaminocarbonyle, N-cyclohexylméthylaminocarbonyle, méthylamino, diméthylamino, diméthylaminocarbonylméthoxy, hydroxyméthyle, hydroxyéthyle, méthylcarbonyle, aminocarbonyle ou méthoxyméthyle

ou lorsque $R_1$ et $R_2$ représentent chacun un atome d'hydrogène, également un groupe hydroxy ou hydroxycarbonylméthoxy

ou lorsque $R_1$ représente un groupe alcoyle ou cycloalcoyle et $R_2$ un atome d'hydrogène ou un groupe méthyle, également un atome de chlore ou de brome, un groupe méthyle, trifluorométhyle, nitro, amino ou aminocarbonyle et

$R_4$ représente un atome d'hydrogène ou, lorsque $R_1$ et $R_2$ représentent chacun un atome d'hydrogène et $R_3$ en position 2 un groupe hydroxy, un atome de chlore ou de brome, également un atome de chlore ou de brome, un groupe hydroxy ou méthoxy,

et leurs sels d'addition d'acides.

3. Nouveaux composés de formule générale I selon la revendication 1, dans laquelle

$R_1$ représente un atome d'hydrogène, un groupe alcoyle avec 1 à 3 atomes de carbone ou un groupe cycloalcoyle avec 4 à 7 atomes de carbone,

$R_2$ représente un atome d'hydrogène, ou lorsque $R_1$ représente un groupe méthyle, également un carbonyle méthyle,

$R_3$ en position 2 représente un atome de chlore ou de brome, un groupe méthyle, trifluorométhyle, hydroxyméthyle, hydroxyéthyle, méthylamino, diméthylamino, cyano ou méthylcarbonyle et

$R_4$ représente un atome d'hydrogène,

et leurs sels d'addition d'acides.

4. La 4'-amino-2-méthyl-4-n-propylamino-diphénylsulfone et ses sels d'addition d'acides.

5. La 4'-éthylamino-4'-amino-2-chloro-diphénylsulfone et ses sels d'addition d'acides.

6. Sels d'addition d'acides physiologiquement supportables composés selon les revendications 1 à 5 avec des acides minéraux ou organiques.

7. Médicament contenant un composé selon les revendications 1 à 5 ou un sel d'addition d'acide physiologiquement supportable selon la revendication 6, conjointement à un ou plusieurs excipients et/ou diluants inertes.

8. Un composé selon les revendications 1 à 5 ou un sel d'addition d'acide physiologiquement supportable de celui-ci selon la revendication 6 pour le traitement des maladies bactériennes, de la malaria ou de la lèpre.

9. Procédé pour la préparation d'un médicament pour le traitement des maladies bactériennes, de la malaria et de la lèpre, caractérisé en ce qu'in composé selon les revendications 1 à 5 ou un sel d'addition physiologiquement supportable de celui-ci selon la revendication 6 est introduit par une voie non chimique dans un ou plusieurs excipients et/ou diluants inertes.

10. Procédé pour la préparation des composés selon les revendications 1 à 6, caractérsé en ce que

a) on réduit un composé de formule générale

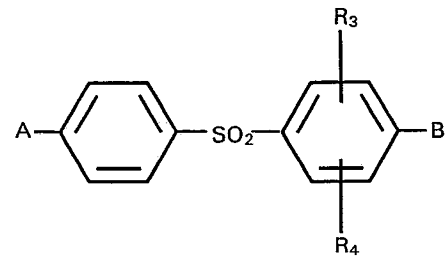

(II)

dans laquelle

$R_3$ et $R_4$ sont définis comme dans les revendications 1 à 5,

l'une des radicaux A ou B représente un groupe nitro et l'autre des radicaux A ou B représente un groupe $\genfrac{}{}{0pt}{}{R_1}{R_2}\!>\!N-$, $R_1$ et $R_2$ étant définis comme dans les revendications 1 à 5, ou également un groupe nitro, ou

b) on clive un ou deux radicaux protecteurs à partir d'un composé de formule générale

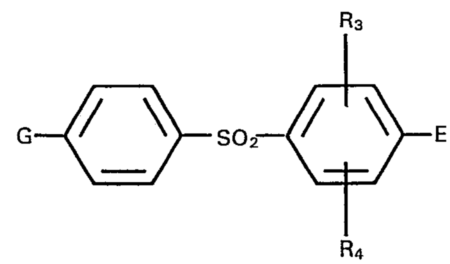

(III)

dans laquelle

$R_3$ et $R_4$ sont définis comme dans les revendications 1 à 5,

E représente un groupe amino, alcoylamino ou cycloalcoylamino protégé par un radical protecteur, la partie alcoyle pouvant contenir 1 à 7 atomes de carbone et la partie cycloalcoyle 3 à 7 atomes de carbone, ou un groupe $\genfrac{}{}{0pt}{}{R_1}{R_2}\!>\!N-$, $R_1$ et $R_2$ étant définis comme dans les revendications 1 à 5, et

G représente un groupe amino éventuellement protégé par un groupe protecteur, au moins l'un des radicaux E ou G devant toutefois représenter l'un des groupes mentionnés plus haut protégé par un radical protecteur, ou

c) pour la préparation de composés de formule générale I, dans laquelle $R_3$ représente un groupe cyano, on déshydrate un composé de formule générale

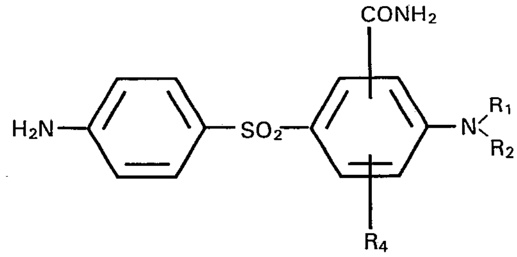

(IV)

dans laquelle

$R_1$, $R_2$ et $R_4$ sont définis comme dans les revendications 1 à 6 ou

d) pour la préparation de composés de formule générale I, dans laquelle $R_3$ représente un groupe hydroxycarbonyl-alcoxy ou dialcoylaminocarbonyl-alcoxy, on alcoyle un composé de formule générale

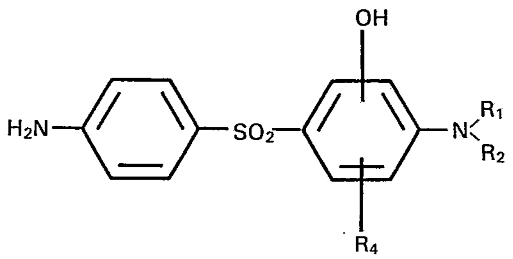

(V)

dans laquelle

$R_1$, $R_2$ et $R_4$ sont définis comme dans les revendications 1 à 5, au moyen d'un composé de formule générale

X - Alk - CO - $R_5$ (VI)

dans laquelle

Alk représente un groupe alcoylène avec 1 à 3 atomes de carbone,

$R_5$ représente un groupe hydroxy, alcoxy ou dialcoylamino, la partie alcoyle pouvant à chaque fois contenir 1 à 3 atomes de carbone, et

X représente un groupe partant nucléophile tel qu'un atome de chlore ou de brome, et éventuellement on hydrolyse ensuite ou

e) pour la préparation de composé de formule générale I, dans laquelle $R_3$ représente un groupe aminocarbonyle, alcoylaminocarbonyle, dialcoylaminocarbonyle ou dialcoylaminocarbonyl-alcoxy, on fait réagir un composé de formule générale

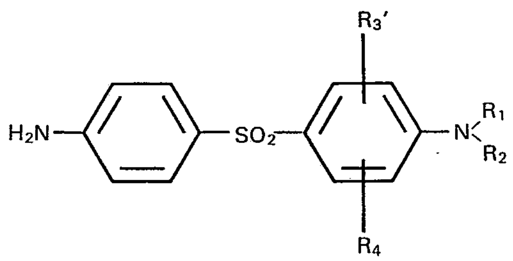

(VII)

dans laquelle

$R_1$, $R_2$ et $R_4$ sont définis comme dans les revendications 1 à 5 et

$R_3'$ représente un groupe hydroxycarbonyle ou hydroxycarbonylalcoxy, ou ses dérivés réactifs avec une amine de formule générale

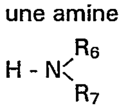

(VIII)

dans laquelle

$R_6$ représente un atome d'hydrogène ou un groupe alcoyle avec 1 à 3 atomes de carbone et

$R_7$ représente un atome d'hydrogène ou un groupe alcoyle avec 1 à 3 atomes de carbone, ou ses réactifs ou

f) pour la préparation de composés de formule générale I, dans laquelle $R_3$ représente un groupe hydroxyméthyle, aminométhyle ou 1-hydroxyalcoyle, on réduit un composé de formule générale

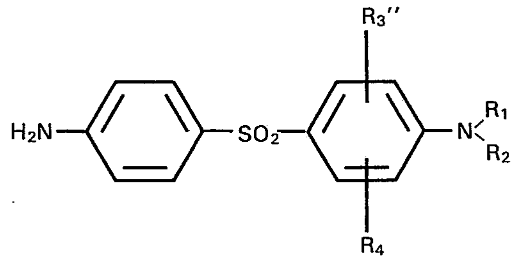

(IX)

dans laquelle

$R_1$, $R_2$ et $R_4$ sont définis comme dans les revendications 1 à 5 et

$R_3''$ représente un groupe hydroxycarbonyle, alcoxycarbonyle, aminocarbonyle ou alcoylcarbonyle, le groupe alcoylcarbonyle pouvant contenir en tout 2 à 4 atomes de carbone, ou

g) pour la préparation de composés de formule générale I, dans laquelle $R_1$ représente un groupe alcoyle ou cycloalcoyle et $R_2$ un atome d'hydrogène, on réduit un composé de formule générale

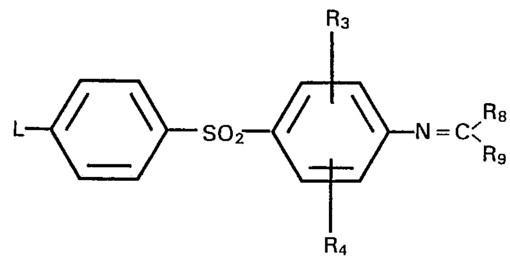

(X)

dans laquelle

$R_3$ et $R_4$ sont définis comme dans les revendications 1 à 5,

L représente un groupe amino ou nitro et

$R_8$ et $R_9$, ensemble avec l'atome de carbone intermédiaire, représentent un groupe alcoylidène avec 1 à 7 atomes de carbone ou un groupe cycloalcoylidène avec 3 à 7 atomes de carbone, ou

h) pour la préparation de composés de formule générale I, dans laquelle au moins l'un des radicaux $R_1$ ou $R_2$ ne représente pas l'hydrogène, et $R_3$ représente un atome d'halogène ou un groupe alcoylamino, dialcoylamino, hydroxyalcoyle, aminoalcoyle, alcoxyalcoyle, hydroxy, amino, trifluorométhyle ou alcoyle, on réduit un composé de formule générale

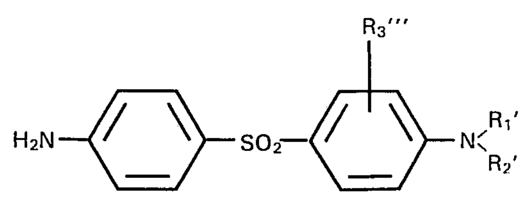

(XI)

dans laquelle

$R_1'$ et $R_2'$ possèdent les significations mentionnées pour $R_1$ et $R_2$ dans les revendications 1 à 5, l'un

des radicaux $R_1'$ ou $R_2'$ devant toutefois représenter un groupe alcanoyle avec 1 à 7 atomes de carbone et

$R_3'''$ représente un atome halogène ou un groupe alcoylamino, dialcoylamino, hydroxyalcoyle, aminoalcoyle, alcoxyalcoyle, hydroxy, amino, trifluorométhyle ou alcoyle, la partie alcoyle pouvant à chaque fois contenir 1 à 3 atomes de carbone et

si on le désire on transforme un composé ainsi obtenu de formule générale I, dans laquelle $R_3$ représente un groupe alcoxycarbonyle ou alcoxycarbonyl-alcoxy, au moyen d'une hydrolyse, en un composé correspondant de formule générale I, dans laquelle $R_3$ représente un groupe hydroxycarbonyle ou hydroxycarbonyl-alcoxy, ou on transforme un composé ainsi obtenu de formule générale I, dans laquelle $R_3$ et/ou $R_4$ représentent un atome de chlore ou de brome, au moyen d'une hydrolyse ou d'une alcoolyse, en un composé correspondant de formule générale I, dans laquelle $R_3$ en position 2 représente un groupe hydroxy ou alcoxy et $R_4$ représente un atome de chlore ou de brome, ou

on transforme un composé ainsi obtenu de formule générale I en son sel d'addition d'acide, en particulier en son sel d'addition d'acide physiologiquement supportable, avec des acides minéraux ou organiques.

11. Médicament contenant un composé selon les revendications 1 à 5 ou un sel d'addition d'acide physiologiquement supportable selon la revendication 6 et un inhibiteur de l'acide dihydrofolique-réductase, conjointement à un ou plusieurs excipients et/ou diluants inertes.

12. Procédé pour la préparation d'un médicament pour le traitement des maladies bactériennes, de la malaria et de la lèpre, caractérisé en ce qu'on introduit un composé selon les revendications 1 à 5 ou un sel d'addition d'acide physiologiquement supportable de celui-ci selon la revendication 6 et un inhibiteur de l'acide dihydrofolique-réductase, par voie non chimique, dans un ou plusieurs excipients et/ou diluants inertes.